# EUROPEAN PATENT APPLICATION

(11) **EP 4 201 431 A1**
(43) Date of publication of application: **28.06.2023**
(21) Application number: 21831644.6
(22) Date of filing: 18.06.2021
(51) Int. Cl.: A61K 47/68, A61K 47/54, A61K 31/337, A61P 35/00, A61P 37/02

(54) **INTERMEDIATE FOR PREPARING ANTIBODY-DRUG CONJUGATE (ADC), PREPARATION METHOD THEREFOR, AND USE THEREOF**

(30) Priority: 28.06.2020 CN 202010601915
(71) Applicant: Dartsbio Pharmaceuticals Ltd., Zhongshan, Guangdong 528400 (CN)
(72) Inventor: WANG, Chunhe, Zhongshan, Guangdong 528400 (CN); SHAO, Ting, Zhongshan, Guangdong 528400 (CN); CHEN, Tianzhi, Zhongshan, Guangdong 528400 (CN); WANG, Qi, Zhongshan, Guangdong 528400 (CN); CHEN, Yili, Zhongshan, Guangdong 528400 (CN); LIN, Yijun, Zhongshan, Guangdong 528400 (CN)
(74) Representative: Gassner, Birgitta
(86) International application number: PCT/CN2021/101061
(87) International publication number: WO 2022/001710

(57) **Abstract**

Provided are an intermediate (connexon and load combination) for preparing an antibody-drug conjugate (ADC), a preparation method therefor, and the use thereof. The connexon and load combination has a structure as represented by formula I. Formula I can be used for coupling highly hydrophobic compound loads, such as paclitaxel (PTX'), and a prepared ADC molecule has good hydrophilicity and thermal stability and can achieve a higher drug-to-antibody ratio.

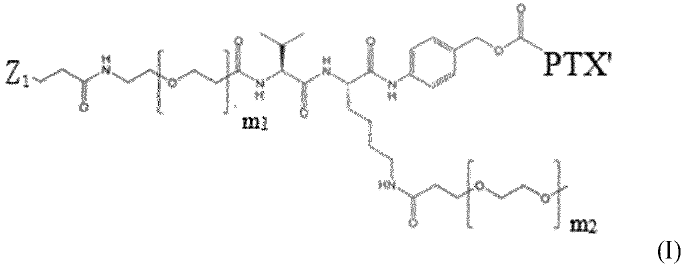

## Description

### Technical field

The invention belongs to the field of biomedicine, and in particular relates to an intermediate (combination of a linker and a load) used for the preparation of an antibody-drug conjugate (ADC) and a preparation method and use thereof.

### Background

Antibody-drug conjugates (ADCs) are a kind of the tumor-targeted therapy. Antibodies targeting tumor antigens are covalently coupled to cytotoxic payloads via chemically synthesized linkers. The choice of drug linkers and payloads plays a key role in the therapeutic efficacy and safety of ADCs.

Currently, most ADC molecules in clinical development stage utilize breakable linkers, including: acid-labile linkers, peptide linkers, and disulfide linkers. A breakable dipeptide linker formed by linking valine-citrulline (Val-Cit) to a self-cleavable spacer benzyl hydroxyl (PAB) is widely used in the clinical development of ADCs. However, due to the strong hydrophobicity, if being combined with a superhydrophobic payload, the VC linker will form a "surfactant-like" structure that is prone to aggregation along with the more hydrophilic part of the antibody, especially when the ratio of the drug to antibody (DARs) is higher than 4. In vivo, the aggregation of ADCs tends to lead to rapid plasma clearance, decreased efficacy and increased toxicity, especially for ADC molecules with high DAR values.

Paclitaxel (PTX), a member of the taxane family, interferes with microtubule assembly during cell division. Although it is effective in many refractory solid tumors, its clinical application is hindered by its poor water solubility. To overcome the solubility problem of PTX, a series of more hydrophilic PTX derivatives and prodrugs have been developed, and PTX-based ADCs are also under development. Since PTX has been used in the treatment of pancreatic cancer and triple-negative breast cancer (TNBC) with high expression of Trop-2, however, so far, the reported PTX-conjugated ADCs have failed to exhibit satisfactory tumor suppression in vivo, and they have not yet entered the clinical development stage.

In summary, there is an urgent need in the field to develop new stable linkers and payloads, so as to obtain ADCs with effective targeting to achieve the highest therapeutic index.

Human trophoblast cell surface antigen 2 (Trop-2) is highly expressed in a variety of human solid tumors, for example, breast cancer, lung cancer, colon cancer, bladder cancer, and pancreatic cancer. However, it is a big challenge to develop a drug using Trop-2 as an ADC target for different expression levels of Trop-2 in key normal tissues such as skin and mucous membranes. IMMU-132 is an ADC molecule formed by linking SN38 to the Trop2-targeting antibody hRS7 via an acid-labile linker with polyethylene glycol. Wherein, the topoisomerase I inhibitor SN38 is the active metabolite of irinotecan, which has moderate cytotoxicity. It has shown positive results in a phase III clinical trial (NCT02574455) in triple-negative breast cancer (TNBC), and is currently approved for marketing. For another ADC molecule targeting Trop-2, RN927C, a more toxic auristatin derivative Aur0101 is used as the payload and linked to antibody via a hydrophobic linker. However, it was terminated in a phase I clinical trial (NCT02122146) due to the high toxicity in vivo, which demonstrates the importance of linkers and payloads in the development of Trop-2-targeting ADC molecules. Up to now, other ADC molecules targeting Trop-2 are still under development. However, whether ADC technology can be successfully applied to the targeted therapy of Trop-2 positive tumors, the key issue is to screen out the best combination of linkers and payloads to achieve the highest therapeutic index. As one of the preferred examples, we used the humanized antibody hRS7 targeting Trop-2 as the ADC antibody part, and screened out the combination of the hydrophilic ADC linker and load of formula I.

### Summary of the invention

The object of the present invention is to provide an ADC containing a taxoid compound as a drug molecule, and an intermediate (i.e., a compound of formula I) that can efficiently couple the taxoid compound to an antibody.

In the first aspect of the present invention, there is provided a compound of formula I, 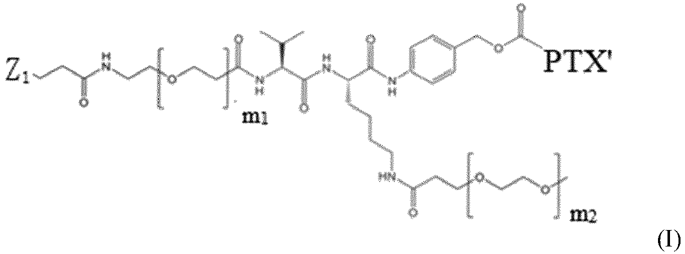 In the formula,
Z₁ is a linker moiety for linking with an antibody;
m₁ is an integer of 0-10;
m₂ is an integer of 10-50;
PTX' is a taxoid compound.

In another preferred example, the Z₁ is a substituted or unsubstituted maleimide group, wherein the substitution means being substituted by a group selected from: D, halogen, OH, C1-C6 alkyl, C1-C6 alkoxy, C1-C6 haloalkyl.

In another preferred example, the Z₁ is linked to the antibody at the 1-position, 3-position or 4-position of the 5-membered ring of maleimide.

In another preferred example, the linking includes: linking to a sulfhydryl group on an antibody, linking to an epsilon-amino group (such as the epsilon-amino group of lysine) on an antibody, linking to a selenocysteine on an antibody, or a combination thereof.

In another preferred example, the Z1 is a chemical linking structure formed by reacting with a naturally occurring active group or an artificially introduced non-natural active group on the antibody.

In another preferred example, a chemical linking structure is formed after the Z₁ is linked to the antibody, and the chemical linking structure is selected from the following group consisting of:
(a) a chemical connection structure formed by a coupling reaction between the sulfhydryl group on the antibody and the maleimides;
(b) a chemical connection structure formed by a coupling reaction between the sulfhydryl group on the antibody and dibromomaleimides;
(c) a chemical connection structure formed by a reaction between the ε-amino group of lysine on the antibody and the active amine group on the linker;
(d) a chemical connection structure formed by a coupling reaction between lysine on the antibody and a maleimide derivative;
(e) a chemical connection structure formed by a coupling reaction between lysine on the antibody and iodoacetamides;
(f) a chemical connection structure formed by a coupling reaction between lysine on the antibody and iminosulfane;
(g) a chemical connection structure formed by a coupling reaction between lysine on the antibody and pyridine disulfide;
(h) a chemical connection structure formed by site-specific coupling with selenocysteine artificially introduced to the antibody;
(i) any combination of the above;

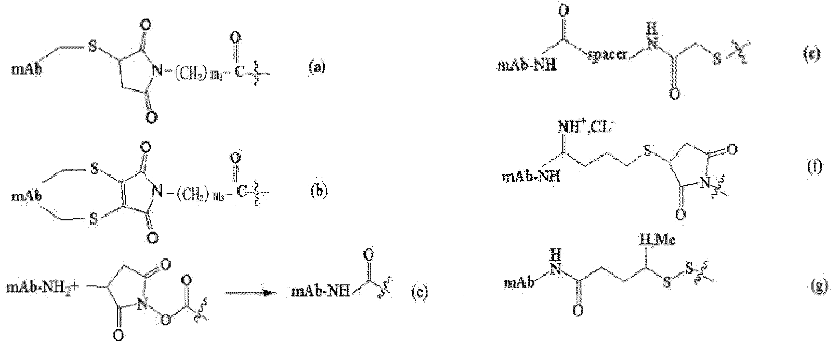

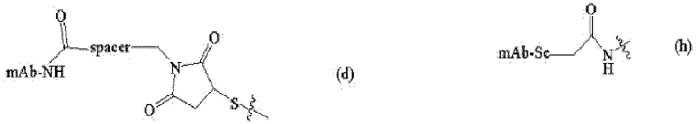

In another preferred example, in the above formulas (a) and (b), m₃ is a positive integer of 1-8 (preferably 2-6).

In another preferred example, in the above formulas (d) and (e), the spacer is selected from: -(CH₂)ₘ₄-, -(CH₂O)ₘ₄-, wherein, m4 is an integer of 0-10 (preferably 1-6).

In another preferred embodiment, the compound of formula I is a compound of formula Ia: 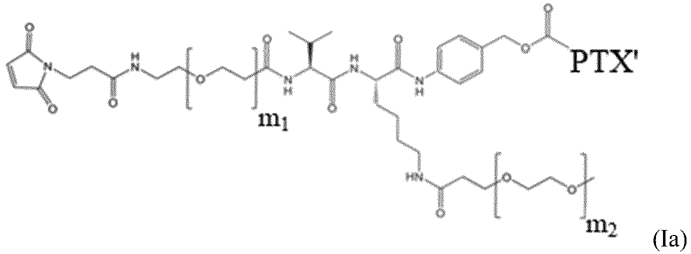 In the formula,
m₁ is an integer of 0-10;
m₂ is an integer of 10-50;
PTX' is a taxoid compound (that is, a group formed from a taxoid compound by removing a H atom).

In another preferred example, m₁ is 0-8, most preferably 0, 4.

In another preferred example, m₂ is 12-40, more preferably 15-35, most preferably 24.

In another preferred embodiment, the taxoid compound is selected from the group consisting of paclitaxel and docetaxel.

In another preferred embodiment, PTX' is paclitaxel (PTX), namely

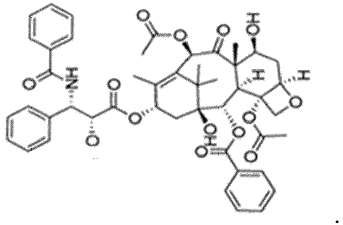

In another preferred example, the compound of formula I has a structure of formula III:

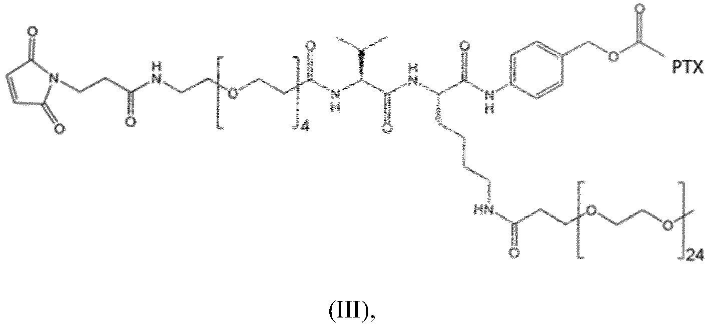

PTX is defined as above.

In the second aspect of the present invention, there is provided an antibody-drug conjugate (ADC), and the antibody-drug conjugate is an antibody-drug conjugate (ADC) formed by coupling the compound of formula I in the first aspect with an antibody.

In another preferred example, the antibody-drug conjugate has the structure of formula II:

Ab-(L-PTX')n II

In the formula,
Ab represents antibody,
L is 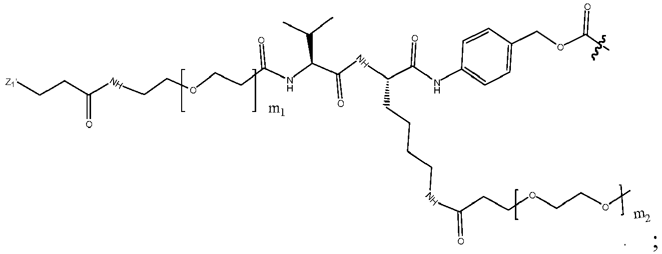 wherein, Z₁' is a group formed from Z₁ group by removing a hydrogen atom;
PTX' is a taxoid compound;
n is the average coupling number of the drug coupled to the antibody;
m₁ and m₂ are defined as above.

In another preferred example, n is an integer or a non-integer of >0, preferably 0.8≤n≤15, most preferably 7-8.

In another preferred example, the antibody is selected from the group consisting of chimeric antibodies, bivalent or bispecific molecules, bispecific antibodies, trispecific antibodies and tetra-specific antibodies.

In another preferred example, the antibody can be an antibody against any target, for example, selected from the following group consisting of:
Trop-2, PD-L1, CTLA4, OX40, EpCAM, EGFR, EGFRVIII, HER2, PTPN2, VEGF, CD11a, CD19, CD20, CD22, CD25, CD30, CD33, CD40, CD56, CD64, CD70, CD73, CD74, CD79, CD105, CD138, CD174, CD205, CD227, CD326, CD340, MUC16, GPNMB, PSMA, Cripto, ED-B, TMEFF2, EphB2, Apo-2, NMP179, p16INK4A, Nectin-4, B7H3, EMA, CEA, MIB-1, GD2, APRIL receptor, folate receptor, mesothelin inhibitor, MET and TM4SF1.

In another preferred example, in the antibody-drug conjugate, m₁ is an integer from 0 to 6.

In another preferred example, in the antibody-drug conjugate, m₂ is an integer of 20, 21, 22, 23, 24, 25, or 26.

In another preferred example, in the antibody-drug conjugate, m₁ is 4 and m₂ is 24.

In another preferred example, in the antibody-drug conjugate, m₁ is 0 and m₂ is 24.

In another preferred example, the antibody is hRS7.

In another preferred example, the antibody-drug conjugate is hRS7-VK-PTX.

In the third aspect of the present invention, there is provided a pharmaceutical composition, which comprises: (a) the antibody-drug conjugate as described in the second aspect, and (b) a pharmaceutically acceptable carrier.

In the fourth aspect of the present invention, there is provided use of the antibody-drug conjugate as described in the second aspect or the pharmaceutical composition as described in the third aspect for the preparation of drugs for treating tumors or immune diseases.

In the fifth aspect of the present invention, there is provided a method for preparing the antibody-drug conjugate as described in the second aspect, the method comprises the steps of:
(1) providing a reaction system, which includes the antibody to be coupled and the compound of formula I in the first aspect;
(2) In the reaction system, carrying out a coupling reaction between the antibody and the compound of formula I to prepare the antibody-drug conjugate as described in the second aspect.

In another preferred embodiment, the pH of the reaction system is 6.0-8.0; preferably, it is 6.0-7.0.

In another preferred example, the molar ratio of the antibody to the compound of formula I (or the taxoid compound) is 1:1-1:20; preferably, 1:6-1:15, more preferably, 1:8-1:12.

In another preferred example, the reaction time is 1 h - 48 h; preferably, 3 h - 36 h.

In another preferred example, the coupling includes site-specific coupling and random coupling.

In another preferred example, the site-specific coupling includes two coupling manners of K-Lock and C-Lock. In the K-Lock coupling manner, VK-PTX' is coupled to the lysine (K) residue in the antibody sequence, and in the C-Lock coupling manner, VK-PTX' is coupled to the cysteine (C) residue in the antibody sequence.

In a sixth aspect of the present invention, there is provided a compound of formula IV:

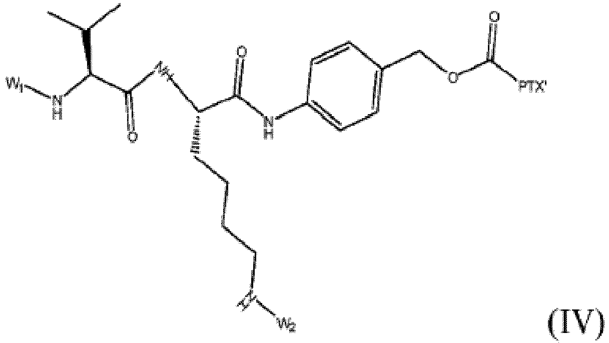

In the formula, W₁ is selected from: H, Fmoc-, 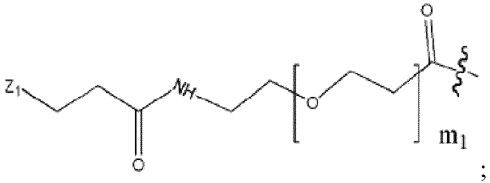
W₂ is selected from: H, Trt-, 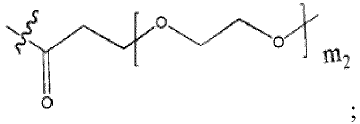
PTX', m₁, m₂ and Z₁ are defined as above.

In the seventh aspect of the present invention, there is provided a method for preparing the compound of formula IV as described in the sixth aspect, comprising the following steps:
(S1) In an inert solvent, in the presence of a base, compound 1 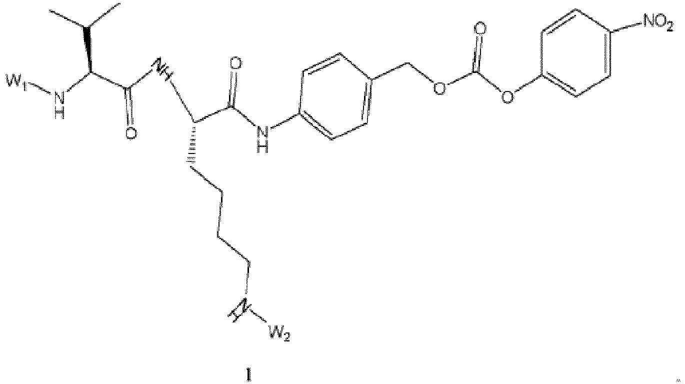 is reacted with
PTX'-H to obtain the compound of formula IV;
PTX', W₁ and W₂ are defined as above.

In another preferred embodiment, in step (S1), the inert solvent is DMF.

In another preferred embodiment, in step (S1), the base is DIEA.

In the eighth aspect of the present invention, there is provided use of the compound of formula I as described in the first aspect for preparing an antibody-drug conjugate.

In another preferred example, the antibody-drug conjugate has the structure of formula II:

Ab-(L-PTX')n II

In the formula,
Ab represents antibody,
L is 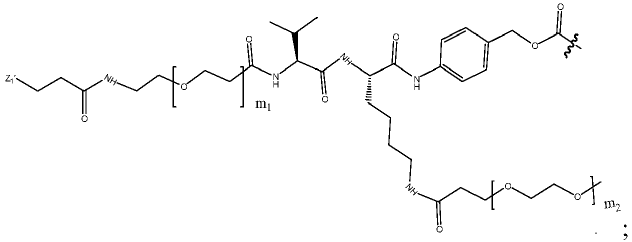 wherein, Z₁' is a group formed from Z₁ group by removing a hydrogen atom;
PTX' is a taxoid compound;
n is the average coupling number of the drug coupled to the antibody;
m₁ and m₂ are defined as above.

In another preferred example, n is an integer or a non-integer of >0, preferably 0.8≤n≤15.

In another preferred example, n is an integer of 4-12 or a non-integer positive number.

In another preferred example, n is an integer of 5-10 or a non-integer positive number; preferably, an integer of 6-9 or a non-integer positive number; more preferably, an integer of 7-8 or a non-integer positive number.

In another preferred example, the antibody is hRS7.

In another preferred example, the antibody hRS7 has the following characteristics:
(a) the amino acid sequence of the light chain variable region of the antibody hRS7 comprises or is the sequence shown in SEQ ID NO.:1; and/or
(b) the amino acid sequence of the heavy chain variable region of the antibody hRS7 comprises or is the sequence shown in SEQ ID NO.:2.

In another aspect, there is provided a method for treating or preventing tumors, the method comprises the step of: administering the above-mentioned antibody-drug conjugate or the above-mentioned pharmaceutical composition to a subject in need.

In another preferred example, the subject is a mammal, including a human.

In another preferred example, the dosage of the antibody-drug conjugate is 0.3-50 mg/kg; preferably, 1-15 mg/kg; more preferably, 8-10 mg/kg .

It should be understood that within the scope of the present invention, the above-mentioned technical features of the present invention and the technical features specifically described in the following (such as example) can be combined with each other to form new or preferred technical solutions. Due to space limitations, they are not repeated herein.

### Description of Figures

Figure 1a shows the molecular structures of VC (Val Cit PAB) linker, VK (Val Lys PAB) linker and hRS7-VK-PTX linker.
Figure 1b shows the ratio of drug to antibody (DAR) in different ADC molecules analyzed by RP-HPLC.
Figure 1c shows that ADC molecules inhibit the growth of transplanted tumors.
Figure 1d shows the single intravenous dose acute toxicity test of hRS7-VK-PTX.
Figure 1e shows the bystander effect validation experiment.
Figure 1f shows endocytosis and localization of anti-Trop-2 ADC detected under confocal microscopy.
Figure 2 shows the molecular structural formulas of hRS7-VK-MMAE and hRS7-VK-SN38.
Figure 3 shows the detection of relevant characteristics of anti Trop-2-ADCs.
Figure 4 shows the expression level of Trop-2 in different tumor cell lines analyzed by flow cytometry.
Figure 5 shows the anti-tumor efficacy of different payloads in different tumor cell lines detected by CCK-8.
Figure 6 shows the anti-tumor efficacy of different ADC molecules in different tumor cell lines detected by CCK-8.
Figure 7 shows that ADC molecules inhibit the growth of tumor in the mouse transplanted tumor model.
Figure 8 shows the comparison of tumor tissues dissected from mouse transplanted tumor models treated with different ADC molecules.
Figure 9 shows the body weight changes of mice treated with ADC.
Figure 10 shows the anti-tumor activity of Trop-2-ADC and its "bystander effect".
Figure 11 shows the single dose acute toxicity test of hRS7-VK-MMAE.

### Detailed Embodiments

The inventor obtained a highly efficient, stability and hydrophobicity-optimized compound of formula I through extensive and in-depth research and a large number of screening, which can be used to prepare targeted antibody-drug conjugates (ADC). The introduction of compound of formula I in ADC can solve the problem of using PTX' as ADC payload. The experimental results show that ADC molecules prepared by using the compound of formula I and the antibody show good efficacy and safety in vivo and in vitro. On this basis, the invention is completed.

Experiments show that the compound of formula I of the invention is a new class of stable linkers and payloads, especially suitable for efficiently coupling drug molecules (such as PTX compounds) that are difficult to form ADC to antibodies, so as to form stable and effective ADCs. The ADC prepared based on the intermediate of the invention has targetability, can achieve excellent therapeutic index, and can significantly reduce the toxic and side effects of ADC on normal cells or tissues.

### Terms

"DAR value" means the drug antibody ratio.

In the present invention, "bystander effect" means that after the small molecules released by ADC cause cytolysis, the payload of ADC is released from the target cell, and then enters other cells around which the target antigen is expressed or not, "bystander killing" will occur. When antigen is highly heterogeneous expressed in tumor tissue, "bystander killing" is necessary.

In the invention, when "a taxoid compound" is used as a part of other compounds, it is the group of the taxoid compound, that is, the group formed from the taxoid compound by removing a H atom.

### Active ingredient

"Compound of Formula I", "intermediate of antibody-drug conjugate", "combination of linker and load" and "linker and payload" are interchangeable.

The invention provides a compound of formula I with the following structure 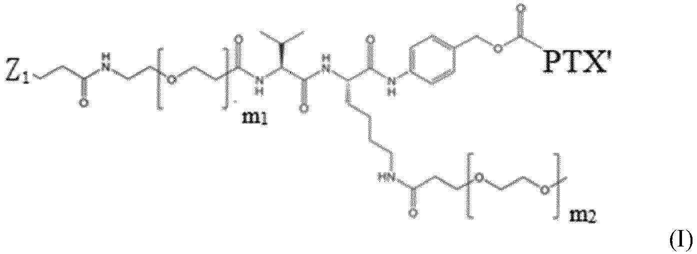 In the formula,
Z₁, m₁, m₂ and PTX are defined as above.

In another preferred example, m₁ is an integer 0-10;
PTX is a taxoid compound.

In another preferred example, m₁ is an integer 0-10; for example, 0-8, more preferably 0-4, most preferably 0 or 4.

In another preferred example, m₂ is 10-50, for example, m₂ is 12-40, more preferably 15-35, and most preferably 24.

Preferably, m₁ is an integer of 1-10 (i.e., 1, 2, 3, 4, 5, 6, 7, 8, 9 or 10);

Preferably, m₂ is an integer of 15-30 (i.e. 15, 16, 17, 18, 19, 20, 21, 22, 23, 24, 25, 26, 27, 28, 29 or 30);

Preferably, PTX is a taxoid compound (such as paclitaxel, docetaxel and natural hydrophilic taxanes).

Preferably, the compound of formula I is a compound of formula Ia:

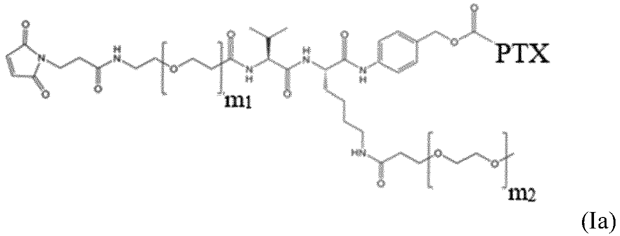

In the formula, m₁, m₂ and PTX are defined as above.

Preferably, the structure of compound of formula I is shown in formula III

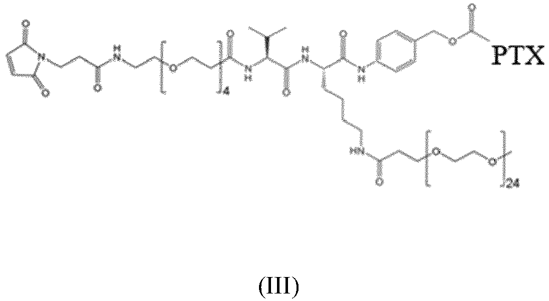

In the formula, PTX is defined as above.

### Antibody

As used herein, the term "antibody" or "immunoglobulin" refers to an isotetrasaccharide protein of about 150,000 daltons with the same structural characteristics, which consists of two identical light chains (L) and two identical heavy chains (H). Each light chain is connected to the heavy chain by a covalent disulfide bond, the number of disulfide bonds between the heavy chains is different for different immunoglobulin isoforms. Each heavy and light chain also has regularly spaced intra-chain disulfide bonds. Each heavy chain has a variable region (VH) at one end, which is followed by a number of constant regions. Each light chain has a variable region (VL) at one end and a constant region at the other end; the constant region of the light chain is opposite to the first constant region of the heavy chain, and the variable region of the light chain is opposite to the variable region of the heavy chain. Special amino acid residues form an interface between the variable regions of light and heavy chains.

As used herein, the term "variable" means that some parts of the variable region in the antibody are different in sequence, giving the binding and specificity of various specific antibodies to their specific antigens. However, the variability is not evenly distributed throughout the antibody variable region. It is concentrated in three fragments in the light chain and heavy chain variable region, called as complementary determinant region (CDR) or hypervariable region. The more conservative part of the variable region is called as the framework region (FR). Each of the variable regions of natural heavy chain and light chain contains four FR regions, which are roughly in β - sheet configuration, connected by three CDRs forming a link ring, and in some cases they can form partial β- sheet configuration. The CDRs in each chain lie closely with one another via the FR regions and form the antigen binding site of the antibody together with the CDRs in another chain (see Kabat et al., NIH Public. No. 91-3242, Volume I, pp. 647-669 (1991)). Constant regions are not directly involved in the binding of antibodies to antigens, but they exhibit different effector functions, for example, participating in antibody-dependent cytotoxicity of the antibody.

The "light chain" of vertebrate antibody (immunoglobulin) can be classified into one of two distinct categories (called as κ and λ) according to the amino acid sequences of their constant regions. According to the amino acid sequence of their heavy chain constant region, immunoglobulins can be divided into different types. There are mainly five classes of immunoglobulins: IgA, IgD, IgE, IgG and IgM, some of which can be further divided into subclasses (homotype), such as IgG1, IgG2, IgG3, IgG4, IgA and IgA2. The constant regions of heavy chains for different immunoglobulins are called as α, δ, ε, γ, and µ, respectively. The subunit structure and three-dimensional configuration of different immunoglobulins are well known to those skilled in the art.

In general, the antigen binding properties of the antibody can be determined by three specific regions located in the variable regions of the heavy chain and light chain, called as variable regions (CDR), which are separated by four frame regions (FR). The amino acid sequences of the four FRs are relatively conservative and do not directly participate in the binding reaction. These CDRs form a ring structure, and are close to each other in spatial structure via β-sheets formed by the FRs therebetween, and the CDR in the heavy chain and the CDR in the corresponding light chain constitute the antigen binding site of the antibody. The amino acid sequence of the same type of antibody can be compared to determine which amino acids constitute the FR or CDR region.

The invention not only includes complete antibodies, but also includes fragments of antibodies with immune activity or fusion proteins formed by antibodies and other sequences. Therefore, the invention also includes fragments, derivatives and analogues of antibodies.

In the invention, the antibody of the invention also includes its conservative variant, which means polypeptides formed by replacing at most 10, preferably at most 8, more preferably at most 5, and preferably at most 3 amino acids of the amino acid sequence of the antibody of the invention with amino acids having similar properties. These conservative mutant peptides are preferably produced by amino acid substitutions in Table A.

**Table A**

| Original residue | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

The sequence of the DNA molecule of the antibody of the present invention or its fragment can be obtained by conventional techniques, such as PCR amplification or genome library screening. In addition, the coding sequences of the light chain and heavy chain can also be fused together to form a single chain antibody.

Once the relevant sequences are obtained, they can be obtained in large quantities by recombination. This is usually done by cloning them into vectors, transferring the vectors into cells, and then isolating relevant sequences from the proliferated host cells by conventional methods.

In addition, relevant sequences can also be synthesized by artificial synthesis, especially when the fragments are short in length. Generally, long sequence fragments can be obtained by synthesizing several small fragments firstly and then connecting them.

At present, it is possible to obtain DNA sequences encoding the antibodies (or fragments thereof, or derivatives thereof) of the invention completely by chemical synthesis. The DNA sequence can then be introduced into various existing DNA molecules (or vectors, for example) and cells known in the art. In addition, the mutation can also be introduced into the protein sequence of the invention by chemical synthesis.

The invention also relates to a vector comprising the above described appropriate DNA sequences and appropriate promoters or control sequences. These vectors can be used to transform appropriate host cells to enable them to express proteins.

Host cells can be prokaryotic cells, such as bacterial cells; or lower eukaryotic cells, such as yeast cells; or higher eukaryotic cells, such as mammalian cells.

In general, the transformed host cells are cultured under conditions suitable for the expression of the antibody of the invention. Then, the antibody of the invention is purified by conventional purification steps of immunoglobulin, such as protein A-Sepharose, hydroxyapatite chromatography, gel electrophoresis, dialysis, ion exchange chromatography, hydrophobic chromatography, molecular sieve chromatography or affinity chromatography, which are conventional separation and purification means well known to those skilled in the art.

The obtained monoclonal antibody can be identified by conventional techniques. For example, the binding specificity of monoclonal antibodies can be determined by immunoprecipitation or in vitro binding tests (such as radioimmunoassay (RIA) or enzyme-linked immunosorbent assay (ELISA)). The binding affinity of monoclonal antibody can be determined, for example, by Scatchard assay of Munson et al., Anal Biochem., 107:220 (1980).

The antibody of the invention can be expressed in cells, or on cell membranes, or secreted out of cells. If necessary, the recombinant protein can be isolated and purified by various separation methods based on its physical, chemical and other properties. These methods are well known to those skilled in the art. Examples of these methods include, but are not limited to: conventional renaturation treatment, treatment with protein precipitation reagent (salting out method), centrifugation, osmosis bacteria disruption, ultrasonic treatment, ultracentrifugation, molecular sieve chromatography (gel filtration), adsorption chromatography, ion exchange chromatography, high-performance liquid chromatography (HPLC) and other various liquid chromatography technologies and combinations of these methods.

In another preferred example, the antibody is hRS7.

In another preferred example, the amino acid sequence of the light chain variable region (V-Kappa) of the antibody hRS7 is the amino acid sequence shown in SEQ ID No.: 1 (DIQLTQSPSLSSASVGDRVSITCKASQDVSIAVAWYQKPGKAPKLLIYS ASYRYTGVPDRFSGSGTDFTLTISSLQPEDFAWYYCQQ).

In another preferred example, the amino acid sequence of the heavy chain variable region (VH) of the antibody hRS7 is the amino acid sequence shown in SEQ ID No.: 2 (QVQLQQSGSELKKPGKVSKASGYTFTNYGMNWVKQAPGQGLKWMGWI NTYTGEPTYTDDFKGRF AFSLDTSVSTA YLQISSLKADDTA VYFCARGGFGSSY WYFDVWGQGSLVTVSS).

### Antibody-drug conjugate and preparation thereof

As used herein, the terms "antibody-drug conjugate of the invention", "antibody drug coupling product of the invention", " conjugate of the invention" or "ADC of the invention" are interchangeable, and refer to the antibody-drug conjugate with the structure of Formula II.

In the invention, the antibody-drug conjugate has the structure of Formula II:

Ab-(L-PTX')n II

In the formula,
Ab represents antibody, which can be any kind of antibody as described above (such as chimeric antibody (e.g. humanized mouse antibody), bivalent or bispecific molecule (e.g. bispecific antibody), bispecific antibody, trispecific antibody and tetra-specific antibody);
PTX ', L, n, m₁, m₂ are defined as above.

Preferably, in Formula II, n is an integer of 4-12 or a non-integer positive number, and preferably, n is an integer of 5-10 or a non-integer positive number; Preferably, it is an integer of 6-9 or a non-integer positive number; More preferably, it is an integer of 7-8 or a non-integer positive number.

Preferably, in the above Formula II, m₁ is an integer of 0, 1, 2, 3, 4, 5 or 6; m₂ is an integer of 20, 21, 22, 23, 24 or 25.

Preferably, in the above Formula II, m₁ is 4; m₂ is 24.

### Coupling method

The invention provides a coupling method of coupling the compound of formula I to the antibody, which greatly improves the killing effect of the antibody on tumor cells without changing the affinity of the antibody.

Typical coupling methods applicable to the invention include C-Lock coupling manner. In the C-Lock coupling manner, the drug molecule is coupled to the cysteine (C) residue in the antibody sequence.

Preferably, the preparation method of the antibody-drug conjugate of the invention is as follows:
a certain amount (e.g. 0.5 mg) of antibody is added with a certain amount (e.g. 8 equivalent) of tris(2-carboxyethyl) phosphine (TCEP), and reacted for a period of time (e.g. 2.5 hours) at a suitable temperature (e.g. 37 °C). Then, the coupling reaction is carried out (e.g. reacted overnight, e.g. for 17 hours at 4 °C) by directly adding the compound of formula I (e.g. 15 equivalent), so as to obtain the ADC of the invention. Finally, the buffer solution is replaced with PBS solution of pH 7.0 by using Amicon Ultra 4 10K ultrafiltration tube.

Preferably, the preparation method of the antibody-drug conjugate of the invention is as follows:
A certain amount (e.g. 0.5 mg) of antibody is added with a certain amount (e.g. 2 equivalent) of tris (2-carboxyethyl) phosphine (TCEP), and reacted for a period of time (e.g. 2.5 hours) at a suitable temperature (e.g. 37 °C). After the reaction is completed, the reaction mixture is concentrated to less than 0.5 ml by centrifugation ultrafiltration method, and is filled with a coupling solution (75mM NaAc, pH6.5, 1mM DTPA, 10% DMSO), and then concentrated to less than 0.5 ml by centrifugation, which operation is repeated three times. Then, the coupling reaction is carried out (e.g. reacted overnight, e.g. for 17 hours at 4 °C) by directly adding the compound of formula I (e.g. 8 equivalent), so as to obtain the ADC of the invention.

Preferably, the preparation method of the antibody-drug conjugate of the invention is as follows:
a certain amount of antibody (e.g. 0.5 mg) is added with a certain amount of dithiothreitol (DTT) (e.g., 3 equivalent), and reacted for a period of time (e.g. 2 hours) at a suitable temperature (e.g. 37 °C). After the reaction is completed, the reaction mixture is concentrated to less than 0.5 ml by centrifugation ultrafiltration method, and is filled with a coupling solution (75mM NaAc, pH6.5, 1mM DTPA, 10% DMSO), and then concentrated to less than 0.5 ml by centrifugation, which operation is repeated three times. Then, the coupling reaction is carried out (e.g. reacted overnight, e.g. for 17 hours at 4 °C) by directly adding the compound of formula I (e.g. 10 equivalent), so as to obtain the ADC of the invention.

### Pharmaceutical composition and administration route

The present invention also provides a pharmaceutical composition containing the ADC of the present invention, and a method for treating diseases in mammals by using the ADC of the present invention. Preferably, the disease is a tumor, including a tumor with a specific target, such as a Trop-2-targeted tumor, a Her2-targeted tumor, or an EGFR-targeted tumor.

The invention also provides use of the antibody-drug conjugate in the preparation of antitumor drugs.

In the present invention, the pharmaceutical composition includes an effective amount of the ADC according to the present invention (as an active ingredient), and at least one pharmaceutically acceptable carrier, diluent or excipient. During the preparation, the active ingredient is usually mixed with or diluted with an excipient. When the excipient acts as a diluent, solid, semi-solid or liquid materials may be used as the medium for excipients, carriers or the active ingredient. Thus, the composition may be a solution, a sterile injectable solution, or the like.

Suitable excipients include: lactose, glucose, sucrose, sorbitol, mannitol, starch, microcrystalline cellulose, polyvinylpyrrolidone, cellulose, water, etc.; formulations can also include: wetting agents, emulsifiers, preservatives (such as methyl hydroxybenzoate and propyl hydroxybenzoate) and so on. The antitumor drug can be made into unit or multiple dosage forms, each dosage form contains the ADC of the present invention in a predetermined amount calculated to achieve the desired curative effect, and suitable pharmaceutical excipients.

The antitumor drugs can be administered through conventional routes, including (but not limited to): intratumoral, intramuscular, intraperitoneal, intravenous, subcutaneous, intradermal, local administration and the like.

As for the use of the drug, a safe and effective amount of the antibody-drug conjugate is administered to humans, wherein the safe and effective amount is preferably in the range of 0.5-50 mg/kg body weight, more preferably 1-10 mg/kg body weight. Of course, factors such as the route of administration and the health status of the patient should also be considered for the specific dosage, all of which are within the skill of skilled physicians.

In addition, the conjugate of the present invention can also be used in combination with other therapeutic drugs, including (but not limited to): various cytokines, such as TNF, IFN, IL-2, etc.; various tumor chemotherapy drugs, drugs that affect nucleic acid biosynthesis, such as 5-FU and methotrexate and the like; alkylating agents such as nitrogen mustard and cyclophosphamide; drugs that interfere with transcription and inhibit RNA synthesis, such as doxorubicin and actinomycin D; drugs that affect protein synthesis, such as vincristine, camptothecin, and certain hormone drugs, etc.

### Compared with the prior art, the present invention has the following main beneficial effects:

(1) The compound of formula I of the present invention can effectively couple taxoid compounds to antibodies, while the conventional val-cit-PAB linker cannot.
(2) The compound of formula I of the present invention has good hydrophilicity.
(3) The antibody-drug conjugate obtained from the compound of formula I of the present invention has an optimized DAR value and a better loading, so that the ADC of the present invention has a broader therapeutic window.
(4) The antibody-drug conjugate provided by the present invention has significant tumor cell killing activity on tumor cells.
(5) For the first time, the present invention has developed a stable PTX' molecule-containing ADC that can be efficiently coupled to antibodies (such as hRS7 antibody). The ADC has a synergistic therapeutic effect and can significantly enhance the killing activity of PTX' on tumor cells.
(6) The compound of formula I of the present invention can be coupled to various antibodies, and the antibody-drug conjugate formed has an optimized DAR value (such as 2, 4, 6 or 8 or above), so that the ADC of the present invention has higher hydrophilicity and thermal stability.
(7) The antibody-drug conjugate of the present invention has better drug efficacy.
(8) The antibody-drug conjugate provided by the present invention has better pharmacokinetic properties and lower toxic and side effects.

The present invention is further illustrated with reference to the specific embodiment below. It should be understood that these examples are only used to illustrate the present invention and are not intended to limit the scope of the present invention. In the following examples, the experimental methods of which the specific conditions are not specified are usually carried out under conventional conditions, such as conditions described in Sambrook et al., Molecular cloning: A Laboratory Manual, (New York: Cold Spring Harbor Laboratory Press, 1989), or according to the conditions suggested by the manufacturer. Percentages and parts are by weight unless otherwise indicated.

### Materials and Methods

### (a) Cell lines and reagents

MDA-MB-468, BXPC-3, SKBR3, CFPAC-1, SCLC-21H, HCC1806, Capan-1, NCIH2452, MDA-MB-231, COLO205, HT29, NCI-H1688 and SK-MES-1 were provided by Shanghai Cell Bank, China.

DMEM supplemented with 10% newborn calf serum (Thermo Fisher Scientific, Waltham, MA, USA). Cells were incubated at 37°C in a humidified atmosphere of 5% CO₂. PE-labeled goat anti-human IgG marker was purchased from Biolegend (San Diego, USA). Humanized Trop-2 antibody hRS7 was prepared according to US Patent 9931417B2. Cell Counting Kit-8 (CCK8) was purchased from Dojindo Laboratories (Tokyo, Japan). Matrigel (#354234) was purchased from BD Biosciences (San Jose, USA). Paclitaxel (PTX), MMAE and SN38 were synthesized by Levena Biopharma (Suzhou, China). Anti-LAMP-1 (#9091), anti-Clathrin (#4796), anti-GM130 (#12480) and 555-anti-rabbit IgG F(ab')2 fragments were purchased from Cell Signaling Technology (Trask USA, Lane Danvers ). IgG H&L (DyLight^{®} 488) was purchased from Abeam Trading (Shanghai) Company Ltd. Human Trop-2 recombinant protein (6xHis Tag) was purchased from Sino Biological (Beijing, China). Formaldehyde was purchased from Polysciences, Inc. (Cat. No. 18814). Normal Goat Serum #5425 was purchased from Cell Signaling Technology (USA, Trask Lane Danvers). ProLong^{™} Live Antifade Reagent (#P36974) and TMB substrate were purchased from Thermo Fisher Scientific Inc. TCEP (Bond Breaker^{™}) was purchased from Pierce (Rockford, IL, USA). Dimethylsulfoxide (DMSO) and other chemicals were products of Sigma-Aldrich^{®} (Merck KGaA).

### (b) Preparation of ADC and detection of DAR value

The reduction reaction was carried out by adding 6 molar equivalents of TCEP (1 mg/mL) to 8 mg/mL of antibody solution containing 9% sucrose, 10 mM acetate, 0.01% Tween-20, pH 5.0 at 37 °C for 2 hours. After reduction, 20 molar equivalents of the linker payload were added per sulfhydryl group in the antibody and incubated at 4°C for more than 16 hours. The solution mixture was concentrated by centrifugation (4000 rpm, 15 minutes, Thermo Fisher ST40R TX-1 ) with an Amicon Ultracontainer (50,000 MWCO, Millipore Corporation). The concentration of ADC was measured by UV detector (Nanodrop 100, Thermo Fisher Scientific Inc.) at 280 nm. The DAR value of the final product was determined by reverse phase (RP)-HPLC and QTOF mass spectrometer.

### (c) RP-HPLC analysis of DAR value of ADC

RP-HPLC analysis was performed on a 1260 Infinity UHPLC (Agilent Technologies) under the following conditions: 1) Agilent PLRP-S1000Å, 8 µm, 4.6 * 250mm; 2) Mobile phase A: 0.01% trifluoroacetic acid in water (TFA); 3) Mobile phase B: acetonitrile solution containing 0.01% TFA; 4) Gradient program: 0-3min (100%A), 3-25min (100%A-100%B), 25-30min (100%B); 5) Column temperature: 70°C; 6) Injection volume: 15µl (1mg/mL). The degree of hydrophobicity of an ADC molecule is proportional to the amount of conjugated payload and can be reflected by its retention time. Since the drug linker is UV-absorbing, the average number of conjugated drug molecules per antibody molecule in the ADC can be calculated according to the following equation: Average number of conjugated drug molecules = (L0 peak area ratio × 0 + L1 peak area ratio × 1 + H0 peak ratio × 0 + H1 peak ratio × 1 + H2 peak ratio × 2 + H3 peak ratio × 3)/100×2.

### (d) SEC-HPLC analysis of ADC aggregates

The SEC-HPLC method was used to monitor the aggregation and degradation of the ADC. After incubation at 60 °C for 1 h, samples were analyzed on a Thermo MAbPac SEC-1, 5 µm, (7.8 × 300 mm) PIN 088460 using a 1260 HPLC system (Agilent). Mobile Phase: phosphate buffered saline (PBS), 50 mM sodium phosphate and 300 mM sodium chloride, pH 6.8. The column was maintained at 26°C during the separation. The detector was set at 280nm. Flow rate: 0.7mL/min. Sample injection volume: 15 µl.

### (e) SDS-PAGE

Samples were boiled in 1x loading buffer for 10 min, and samples containing 5 µg of hRS7 or ADC were separated by SDS-PAGE (10% Bio-Rad Criterion Tris-HCl gel), then stained with Coomassie brilliant blue for 5 min, and detected with BioRad ChemiDoc MP.

### (1) Detection of Trop-2 expression level of tumor cells

The cells and 5 µg/mL of hRS7 were incubated in ice-cold staining medium on ice for 30 min and washed twice with an ice-cold staining medium to remove unbound antibodies. Cells were then stained with PE goat anti-human IgG (5 µg/mL) in the ice-cold staining medium for 30 minutes and washed twice. Cells were examined by flow cytometry.

### (g) Detection of the binding of antigen to antibody by ELISA

96-well immunoplates were coated with 2 µg/mL of antigenic protein with histidine tag (Sino Biological Inc.) and incubated overnight at 4°C. Plates were washed, blocked with 1% casein, and then incubated with 10 nM antibody or ADC for 1 hour at room temperature. After washing, anti-human κ-HRP (Biolegend San Diego, USA) was added and incubated for 1 hour. After washing, TMB substrate was added and detected with a SpectraMax M5e (Molecular Devices) microplate reader at 450 nm.

### (h) Detection of ADC endocytosis rate

To assess the endocytosis rate of ADC, MDA-MB-231 or CFPAC-1 cells (1 × 10⁶) were incubated with 5 µg/mL of ADC samples in a staining medium for 30 min on ice, and washed twice, and then it was placed in a fresh complete medium. Internalization was initiated in a humidified chamber at 37 °C. After the incubation, cells were washed twice with an ice-cold staining medium and then stained with goat anti-human IgG-PE. Cells were analyzed by flow cytometry to detect the level of ADC remaining on the surface.

### (i) Endocytosis and intracellular localization of ADC detected by confocal microscopy

SKBR3 cells were incubated with 5 µg/mL of hRS7-VK-PTX or hRS7-VK-SN38 in a 24-well plate (1×10⁴ cells/well) in a 5% CO₂, 37°C incubator for 45 or 90 min. Cells were washed once with PBS, fixed and permeabilized. After washing, cells were stained with 488 goat anti-human IgG (H&L) or 555 anti-rabbit IgG F(ab')₂ fragment. Nuclear DNA was stained with 4',6-dimidyl-2-phenylindole (DAPI). Immunofluorescence was recorded with an Olympus BK71 confocal microscope.

### (j) Cell Viability Assay

Cell viability was determined using Cell Counting Kit 8 (CCK-8). Cells were seeded into 96-well plates at 3000-10000 cells/well. After incubation overnight, drugs were added at different concentrations. Plates were incubated in a 5% CO₂, 37°C humidified chamber for 96 hours. CCK8 reagent was added thereto and the plates were placed back to the incubator until the absorbance at 450 nm of untreated control cells was greater than 1.0. Growth inhibition was measured as the percent growth relative to untreated cells. Dose response curve was generated from the mean values of two or three tests, and IC50 values were calculated using Prism GraphPad software.

### (k) Detection of drug efficacy in mice

Five-week-old female athymic BALB/c nu/nu mice were purchased from Charles River Company (Shanghai, China), and mice were randomly divided into 5-6 mice/group. Animal handling and procedures were approved and performed in accordance with the requirements of the Institutional Animal Care and Use Committee (IACUC) of Shanghai Institute of Materia Medica, Chinese Academy of Sciences. All models were inoculated subcutaneously on the side of mice. BXPC-3, HCC1806 and NCIH1688 cells were established by injecting and homogenizing 5 × 10⁶ cells in Matrigel matrix. COLO205 cells were established by injecting and suspending 1 × 10⁷ cells in Matrigel matrix. After the tumor volume reached about 200 mm³, the tumor-bearing mice were randomly divided into treatment groups and a control group according to the tumor volume. Each drug was administered intraperitoneally. Mice were injected at doses of 3 or 10 mg/kg (10 mL/kg). Tumor volume was defined as length × 1/2 × width × width. The percentage of inhibition was calculated by the following formula: (1-average tumor volume of the treatment group/average tumor volume of the control group)×100%. Toxicity of the different treatment groups was assessed by monitoring weight loss.

### (I) Determination of dose tolerability

Dose tolerability assay was performed in BALB/c mice (3 mice per dose). The drug was given in a single intravenous dose. The injection doses of hRS7-VK-PTX were 60, 80 and 100 mg/kg, and the injection doses of hRS7-VK-MMAE were 60 and 80 mg/kg, respectively. Toxicity was assessed by observing mouse behavior, weight loss and survival. The weight change rate (%) was calculated as: (body weight - body weight before treatment)/( body weight before treatment)×100%.

### (m) Statistical Analysis

Prism GraphPad version 6 was used for statistical analysis. Results were shown as mean ± SD. Statistical differences between control and experimental groups were calculated by Student's t-test, and P < 0.05 was considered a significant difference.

### Example 1

### Antibody preparation

In this example, hRS7 antibody was expressed and purified to obtain hRS7 antibody. The method was detailed as below:
The amino acid sequences of hRS7 light and heavy chains were obtained from US Patent 9931417 B2, and then reverse-transcribed into cDNA sequences by Vector NTI software. The DNA sequence was synthesized and subcloned into the pcDNA3.1 vector, which was amplified in E. coli. The purified plasmid was transfected into HEK293 cells with PEI. Cells were then suspended and cultured in Gibco^{®} FreeStyle^{™} 293 Expression Medium. After culturing for 5 days, cell culture supernatant was harvested and antibodies were purified by protein A affinity.

### Example 2

### Preparation of Compound VK-PTX

The structure of compound VK-PTX is shown below:

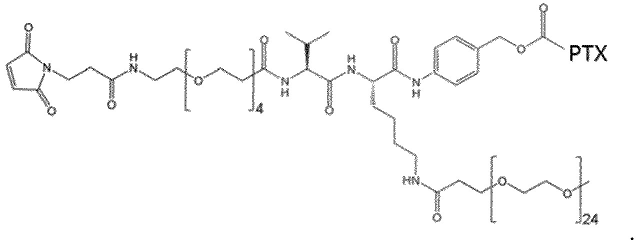

Paclitaxel was reacted with the VK linker to form the compound VK-PTX, i.e., Mal-peg4-Val-Lys(peg24)-PAB-paclitaxel (VK-PTX), the method was as follows:

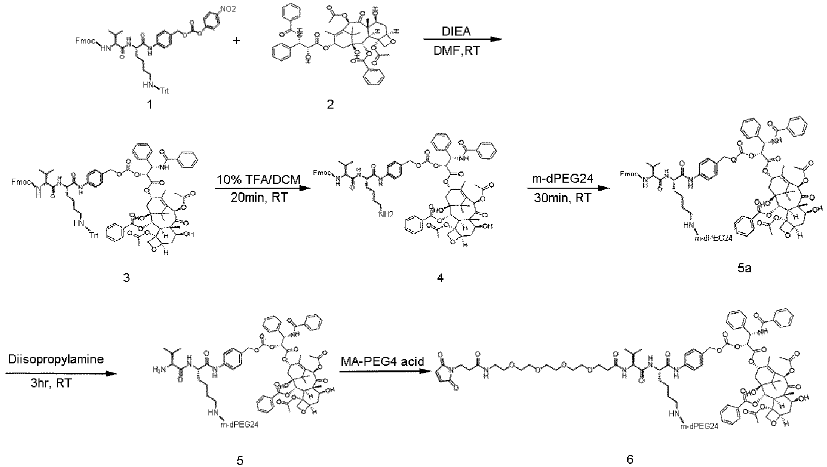

### Step 1: Synthesis of Compound 3 (Fmoc-Val-Lys(Trt)-PAB-PTX)

Fmoc-Val-Lys(Trt)-PAB-PNP (purchased from Levena Biopharma, USA) and PTX (purchased from Levena Biopharma, USA) were dissolved in anhydrous DMF, and DIEA was added to the solution, and the mixture was stirred and reacted at room temperature 22°C for 2 hours, and the target product Fmoc-Val-Lys(Trt)-PAB-PTX was directly purified by reverse HPLC, after lyophilization, a white powder compound 3 was obtained.

### Step 2: Synthesis of compound 4 (Fmoc-Val-Lys-PAB-PTX TFA salt)

Compound 3 obtained in step 1 was dissolved in 10% TFA/DCM solution and stirred at room temperature for 20 minutes. After reaction, the mixture was concentrated under reduced pressure and purified by reverse-phase HPLC to obtain compound 4, Fmoc-Val-Lys-PAB-PTX TFA Salt.

### Step 3: Synthesis of Compound 5

Compound 4 obtained in step 2, m-PEG24 acid and HATU were dissolved in DMF, added with DIEA, and the resultant was stirred at room temperature for 30 minutes to obtain product 5a.

Diisopropylamine was added to the reaction mixture and stirred at room temperature for 3 hours. After the reaction, the mixture was concentrated and purified by reverse HPLC, and lyophilized to obtain a waxy compound 5.

### Step 4: Synthesis of Compound 6 (Mal-peg4-val-Lys(m-PEG24)-PAB-PTX)

Compound 5 obtained in Step 3, m-PEG4-acid and HATU were dissolved in DMF, added with DIEA, and the resultant was stirred for 20 minutes. Thereafter, the desired compound 6, Mal-peg4-val-Lys(m-PEG24)-PAB-PTX (VK-PTX) was finally obtained by reverse HPLC purification and lyophilization.

### Example 3 Preparation of VK-MMAE, VK-SN38

Compounds VK-SN38 and VK-MMAE were prepared in a similar manner.

### (1) Preparation of VK-SN38

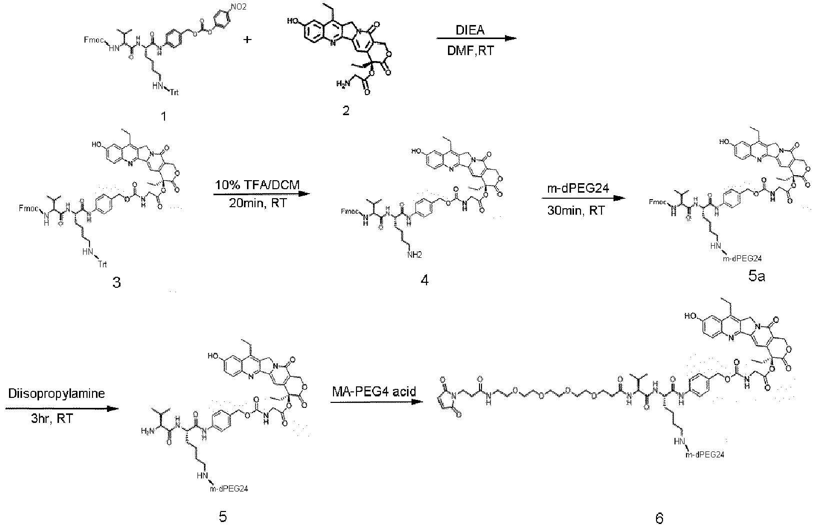

### Step 1: Synthesis of Compound 3

Fmoc-Val-Lys(Trt)-PAB-PNP (purchased from Levena Biopharma, USA) and SN38 (purchased from Levena Biopharma, USA) were dissolved in anhydrous DMF, and DIEA was added to the solution. The mixture was stirred and reacted at room temperature 22°C for 2 hours, and the target product Fmoc-Val-Lys(Trt)-PAB-SN38 was directly purified by reverse HPLC, after lyophilization, a white powder compound 3 was obtained.

### Step 2: Synthesis of Compound 4

Compound 3 was dissolved in 10% TFA/DCM solution and stirred at room temperature for 20 minutes. After reaction, the mixture was concentrated under reduced pressure and purified by reverse-phase HPLC to obtain compound 4, Fmoc-Val-Lys-PAB-SN38 TFA salt.

### Step 3: Synthesis of Compound 5

Compound 4, m-PEG24 acid and HATU were dissolved in DMF, and DIEA was added, the resultant was stirred at room temperature for 30 minutes to obtain product 5 a.

Diisopropylamine was added to the reaction mixture and stirred at room temperature for 3 hours. After the reaction, the mixture was concentrated and purified by reverse HPLC, and lyophilized to obtain a waxy compound 5.

### Step 4: Synthesis of Compound 6

### Compound 4, m-PEG24 acid and HAT

Compound 5, m-PEG4-acid and HATU were dissolved in DMF, DIEA was added and the resultant was stirred for 20 minutes. Thereafter, the desired compound 6: Mal-peg4-val-Lys(m-PEG24)-PAB-SN38 (VK-SN38) was finally obtained by reverse HPLC purification and lyophilization.

### (2) Synthesis of VK-MMAE

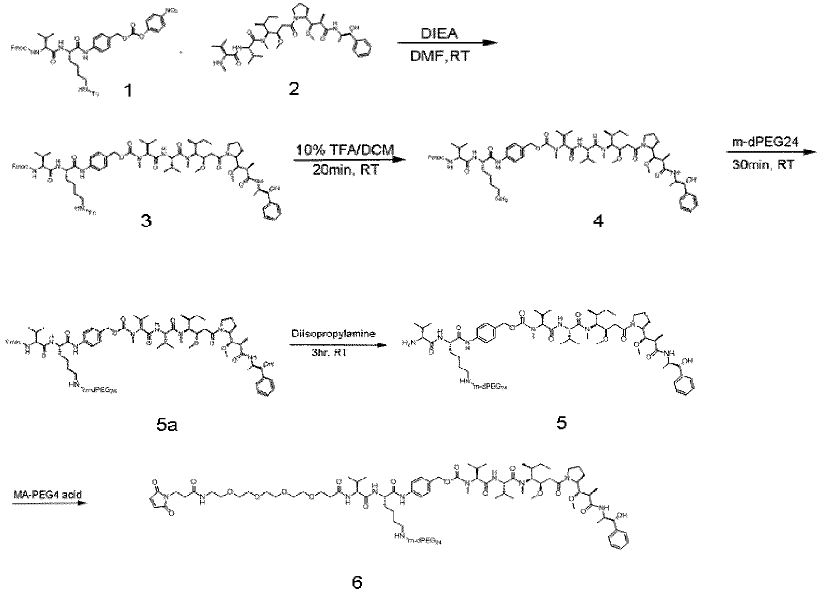

### Step 1: Preparation of compound 3

Fmoc-Val-Lys(Trt)-PAB-PNP (purchased from Levena Biopharma, USA) and MMAE (purchased from Levena Biopharma, USA) were dissolved in anhydrous DMF, and DIEA was added to the solution. The mixture was stirred and reacted at room temperature 22°C for 2 hours, and the target product Fmoc-Val-Lys(Trt)-PAB-MMAE was directly purified by reverse HPLC, after lyophilization, a white powder compound 3 was obtained.

### Step 2: Preparation of compound 4

Compound 3 was dissolved in 10% TFA/DCM solution and stirred at room temperature for 20 minutes. After reaction, the mixture was concentrated under reduced pressure and purified by reverse-phase HPLC to obtain compound 4, Fmoc-Val-Lys-PAB-MMAE TFA salt.

### Step 3: Preparation of compound 5

Compound 4, m-PEG24 acid and HATU were dissolved in DMF, DIEA was added, and the resultant was stirred at room temperature for 30 minutes to obtain product 5a.

Diisopropylamine was added to the reaction mixture and stirred at room temperature for 3 hours. After reaction, the mixture was concentrated and purified by reverse HPLC, and lyophilized to obtain a waxy compound 5.

### Step 4: Preparation of compound 6

Compound 5, m-PEG4-acid and HATU were dissolved in DMF, DIEA was added and the resultant was stirred for 20 minutes. Thereafter, the desired compound 6: Mal-peg4-val-Lys(m-PEG24)-PAB-MMAE was finally obtained by reverse HPLC purification and lyophilization.

### Example 4

### Preparation of ADC

The VK-PTX, VK-MMAE, and VK-SN38 prepared in Examples 2 and 3 were coupled with the antibody prepared in Example 1 respectively by a coupling reaction to form corresponding ADCs.

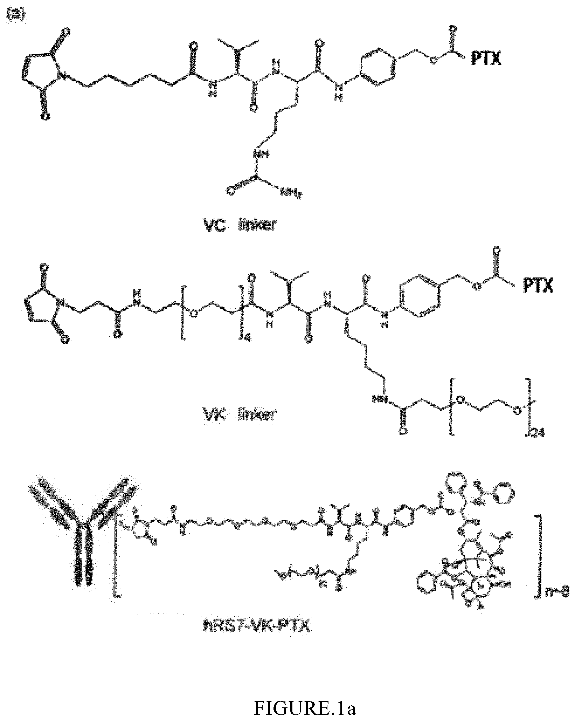

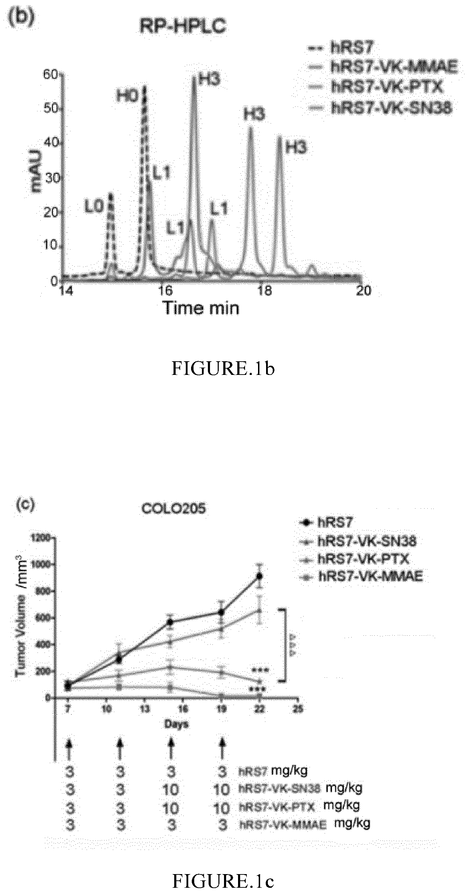

A stable PTX-loaded ADC molecule with a DAR value of 8, named hRS7-VK-PTX (Fig. 1a and b), was successfully prepared via the VK linker.

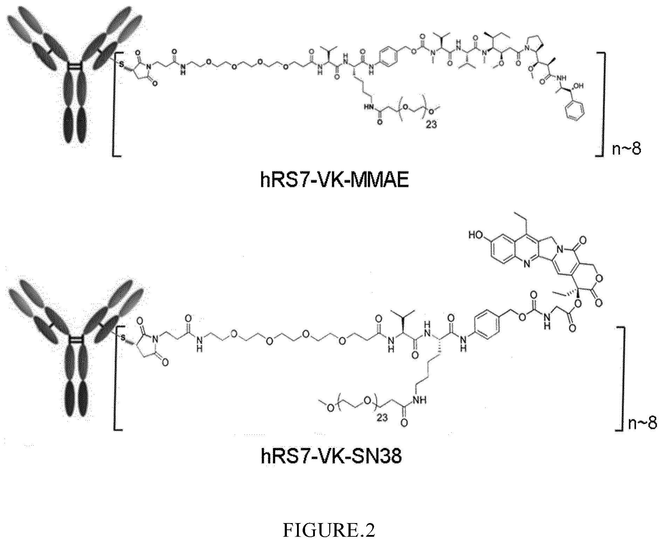

For comparison, Trop-2-ADCs with payloads of MMAE and SN38 with a DAR value of 8, namely hRS7-VK-MMAE and hRS7-VK-SN38, were also prepared (Fig. 1b, Fig. 2).

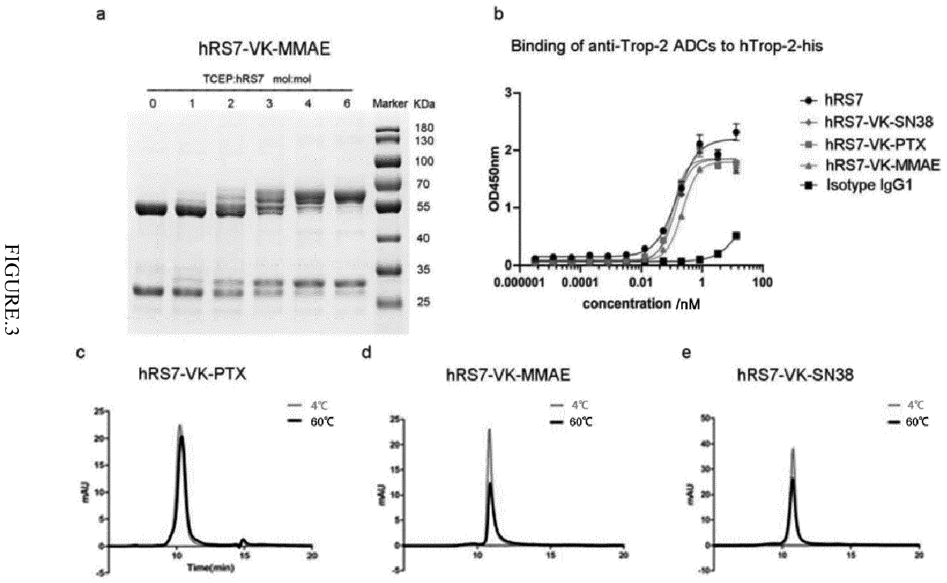

The SDS-PAGE under reducing conditions of hRS7-VK-MMAE with DAR values from 0 to 8 is shown in Fig. 3a. Wherein, MMAE cannot be used alone as a drug for tumor therapy due to its high toxicity, but it has been widely used as a payload of ADC; SN38 is a moderately toxic payload used in the above-mentioned IMMU-132. Both MMAE and SN38 are somewhat hydrophobic, but less than that of PTX. Three Trop2-ADCs prepared with the VK linker did not showed severe aggregation and degradation even after incubation at 60°C for 1 hour (Fig. 3c, d, e), indicating that PEGylation of linkers can significantly improve hydrophobicity and stability of ADC molecules.

The affinity of different ADC molecules with DAR value of 8 to human Trop-2 protein (6xHis tag) is shown in Fig. 3b.

### Comparative example 1

### Preparation of Control ADC Molecules

A Trop-2-targeted hRS7-VC-PTX molecule was prepared via a hydrophobic VC linker (Fig. 1a). However, the molecule completely precipitated out of solution during the preparation process, so its therapeutic ability and safety could not be assessed.

Therefore, it can be seen that conjugating a hydrophobic linker with a superhydrophobic payload will destabilize the ADC molecule, leading to the increase of aggregates and precipitation.

### Example 5

### Efficacy study of hRS7-VK-PTX

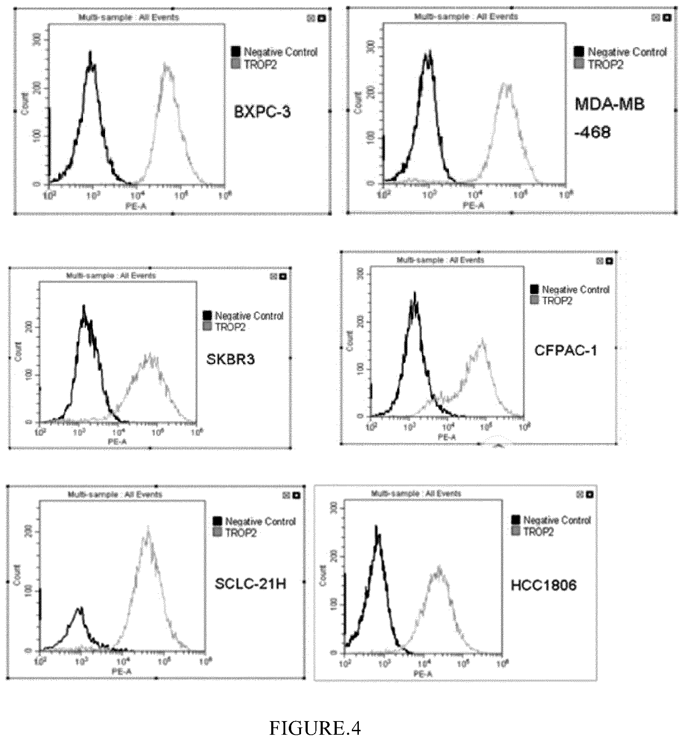

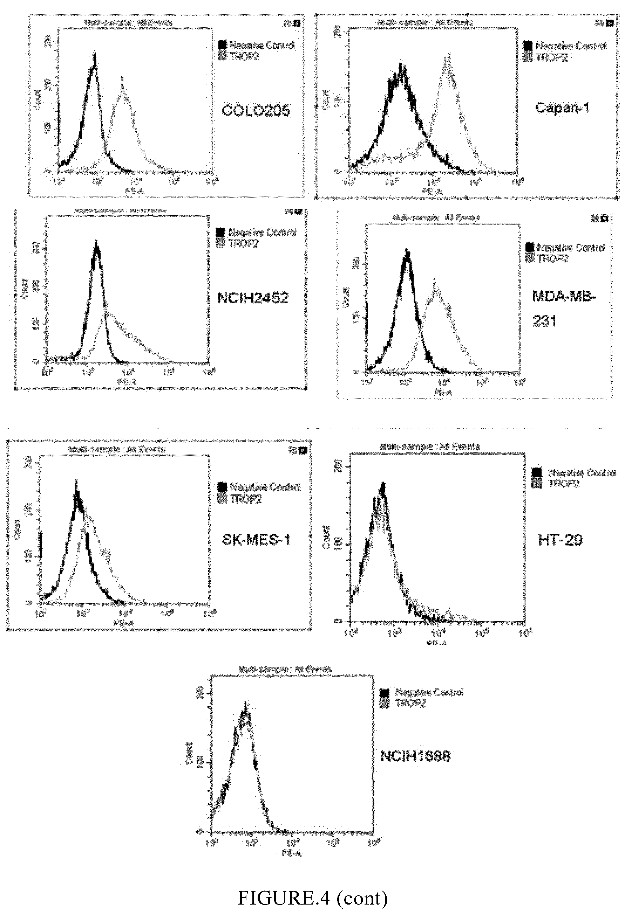

In this example, cancer cells in PBS (1×10⁶ cells/mL) were incubated with 5 µg/mL hRS7 for 30 minutes at 4°C, and human IgG1 was used as an isotype control. The results are shown in Figure 4.

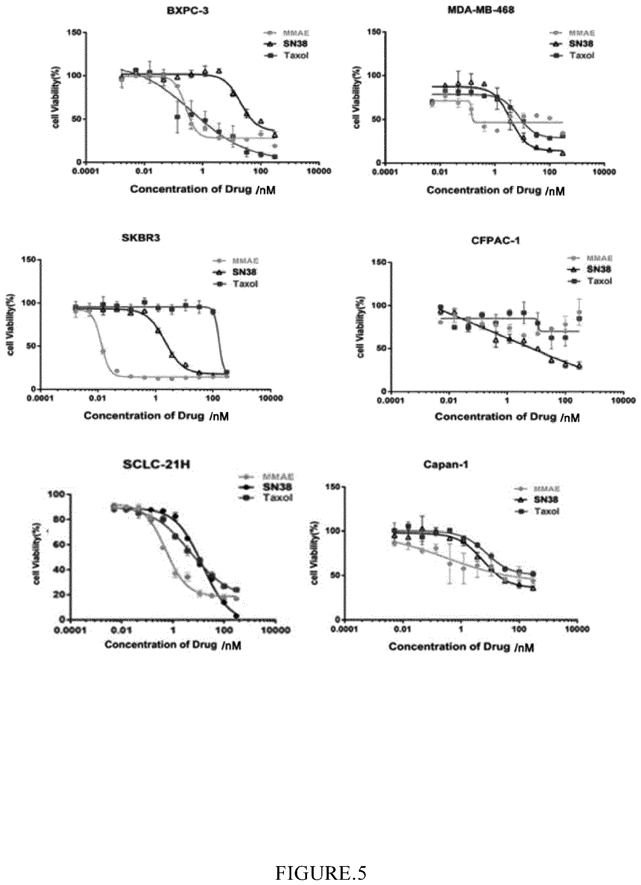

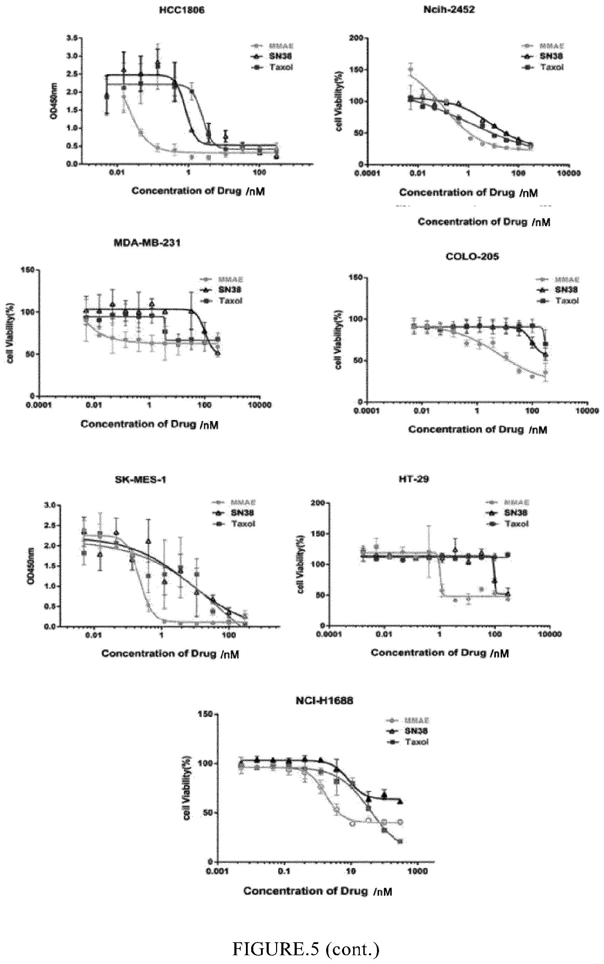

The anti-tumor efficacy of different payloads on different tumor cell lines was detected by CCK-8, and the results are shown in Fig. 5.

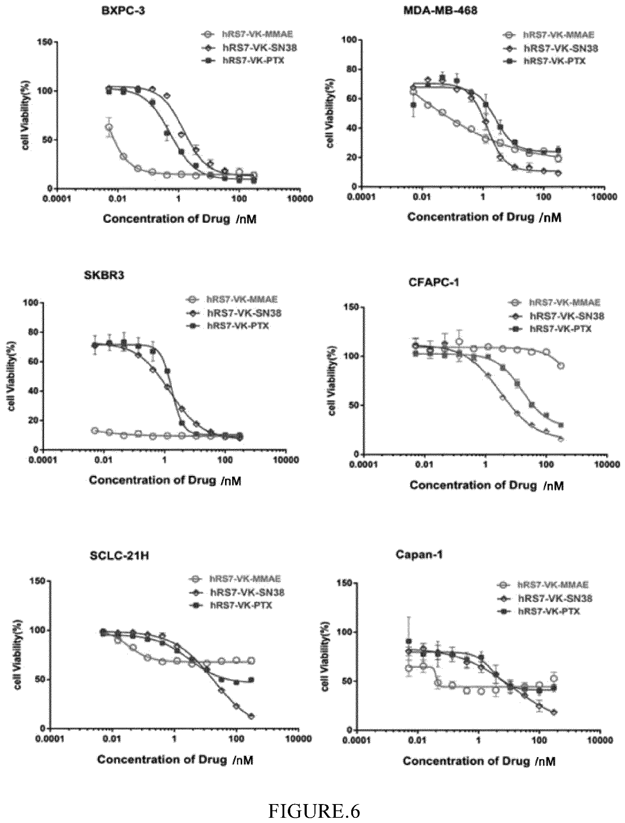

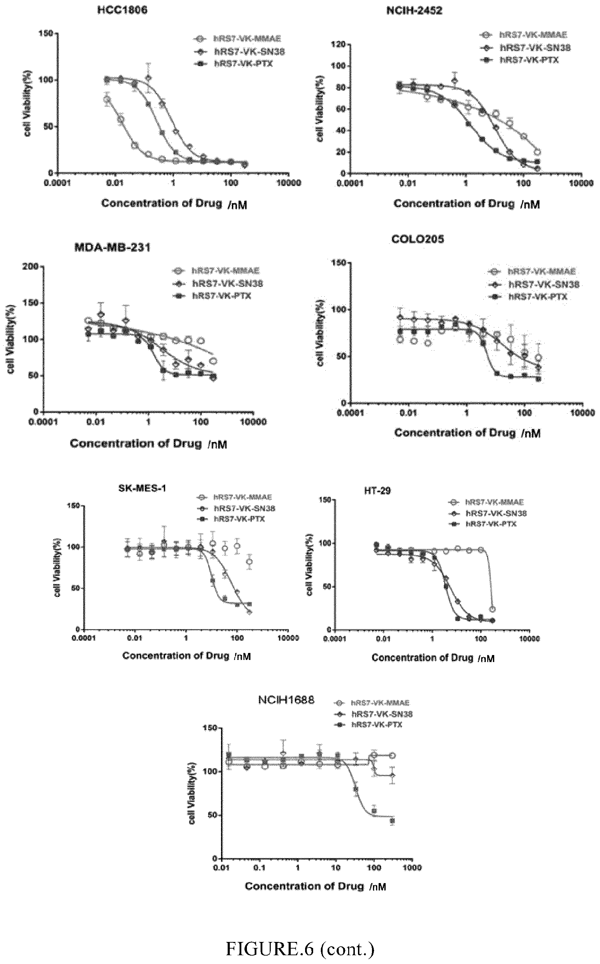

The anti-tumor efficacy of different ADC molecules on different tumor cell lines was detected by CCK-8, as shown in Fig. 6.

In tumor cell lines with different expression levels of Trop-2, the cytotoxicity of individual PTX molecules was weaker than that of MMAE, but close to that of SN38 (Fig. 4, Fig. 5, Fig. 6). However, after ADC was prepared, hRS7-VK-PTX was more effective than hRS7-VK-MMAE and hRS7-VK-SN38 in tumor cell lines with low expression of Trop-2, including COLO205, MDA-MB-231, SK - MES-1, Capan-1, NCIH2452 (see Table 1 and Table 2).

In addition, hRS7-VK-PTX also showed good inhibitory effect in PTX-resistant tumor cell lines, including SKBR3, CFPAC-1, MDA-MB-231 and COLO205 (see Table 1 and Table 2).

**Table 1: Comparison of the efficacy of hRS7-VK-PTX, hRS7-VK-MMAE and hRS7-VK-SN38 on tumor cell lines with different expression levels of Trop-2**

| **Cell name** | **Tumor type** | **TROP2 expression MFI (background)** | | **hRS7-VK-PTX IC50 (95%CI) nmol/L** | **hRS7-VK-SN38 IC50 (95%CI) nmol/L** | **hRS7-VK-MMAE IC50 (95%CI) nmol/L** |
|---|---|---|---|---|---|---|
| BXPC-3 | Pancreas | 64848 | (1073) | 0.5347 (0.4222 to 0.6772) | 1.504 (1.229 to 1.841) | 0.002354 (1.475e-005 to 0.3758) |
| MDA-MB-468 | Mammary | 55877 | (978) | 2653 (1.538 to 4.579) | 1_388 (1.135 to 1.650) | 0.003453 (1.431e-006 to 8.331) |
| SKBR3 | Mammary | 43300 | (1931) | 1743 (1.474 to 2061) | 1.454 (1279 to 1.653) | ∼ 2.243e-006 ((Very wide)) |
| CFPAC-1 | Pancreas | 38400 | (1565) | 16.04 (13.13 to 19.60) | 3.139 (2.262 to 4.357) | >300nM |
| SCLC-21H | Small cell lung cancer | 35100 | (1225) | 2.962 (2.404 to 3.650) | 1897 (1458 to 24.68) | 0.03216 (0.02081 to 0.04970) |
| Capan-1 | Pancreas | 26425 | (3681) | 2.93 (1.267 to 6.775) | 12.39 (2.619 to 58.60) | >300nM |
| HCC1806 | Mammary | 21200 | (692) | 0.2656 (0.2417 to 0.2919) | 0.6769 (0.7023 to 1.095) | 0.01747 (0.01197 to 0.02551) |
| NCIH2452 | Lung | 9946 | (1593) | 1.405 (1.197 to 1.650) | 10.27 (7,697 to 13.69) | - 1.212e+019 (Very wide) |
| MDA-MB-231 | Mammary | 8693 | (1254) | 1.596 (1.139 to 2.234) | 3.001 (0.6342 to 1420) | >300nM |
| COLO205 | Colorectal | 8037 | (811) | 4.92 (4.156 to 5.823) | 16.75 (-0.910 to 5712) | >300nM |
| SK-MES-1 | Lung | 2504 | (891) | 10.11 (8.343 to 12.25) | 60.47 (29.40 to 124.4) | >300nM |
| HT29 | Colorectal | 1790 | (694) | 3479 (3.100 to 3.904) | 5.681 (4.753 to 5.790) | - 280.2 (Very wide) |
| NCI-H1688 | Lung | 657 | (688) | 32.42 (28.08 to 37.43) | >300nM | >300nM |

**Table 2 Inhibitory efficacy of payloads PTX, MMAE and SN38 on cancer cells with different expression levels of Trop-2**

| **Cell name** | **Tumor type** | **TROP2 expression MFI (background)** | | **Paclitaxol IC50 (95% CI) nmol/L** | **SN38 IC50 (95%CI) nmol/L** | **MMAE IC50 (95%CI) nmol/L** |
|---|---|---|---|---|---|---|
| BXPC-3 | Pancreas | 64848 | (1073) | 0.466 (0.1358 to 1.599) | 20.81 (15.38 to 28.20) | 0.266 (0.2241 to 0.3158) |
| MDA-MB-466 | Mammary | 55877 | (978) | 7,225 (4.829 to 10.81) | 3.717 (2.330 to 5.929) | ∼ 01412 (Very wide) |
| SKBR3 | Mammary | 43300 | (1931) | ∼ 162.0 (Very wide) | 2.139 (1.852 to 2.470) | 0.01311 (0.01167 to 0.01448) |
| CFPAC-1 | Pancreas | 38400 | (1585) | > 300nM | ∼0.2635 (Very wide) | >300nM |
| SCLC-21H | Small cell lung cancer | 35100 | (1225) | ∼ 162.0 (Very wide) | 2.139 (1.852 to 2.470) | 0.01311 (0.01187 to 0.01448) |
| Capan-1 | Pancreas | 26425 | (3681) | 7.489 (4.714 to 11.90) | 5.81 (3.657 to 9.229) | 0.3024 (0.001854 to 49.32) |
| HCC1806 | Mammary | 21200 | (692) | 2.468 (1.434 to 4.249) | 0.8422 (0.4813 to 1.474) | 0.0177 (0.0007132 to 0.4394) |
| NCIH2452 | lung | 9946 | (1593) | 1.03 (0.1093 to 9.706) | 7.537 (3.574 to 15.89) | 0.1004 (0.03450 to 0.2920) |
| MDA-MB-231 | Mammary | 8693 | (1254) | > 300nM | 102.6 (49.48 to 212.9) | >300nM |
| COLO205 | Colorectal | 8037 | (611) | > 300nM | 93.37 (53.88 to 161.8) | 6.158 (1.559 to 24.31) |
| SK-MES-1 | Lung | 2504 | (891) | 53.12 (0.003401 to 829764) | 6.959 (0.4174 to 116.0) | 0.2245 (0.1829 to 0.2757) |
| HT29 | Colorectal | 1790 | (694) | > 300nM | >300nM | ∼ 1051 (Very wide) |
| NCI-H1688 | Lung | 657 | (688) | 37.98 (18.09 to 79.76) | 8.4 (6.109 to 11.55) | 1.741 (1.435 to 2.112) |

These results demonstrate that the use of the VK linker in PTX-loaded ADCs significantly enhances PTX's inhibition of tumor cells in vitro. Therefore, the Trop-2-ADC with PTX as the payload shows better in vitro drug efficacy in cell lines with low expression of Trop-2, and overcomes the resistance of PTX single drug to a certain extent.

### Example 6 Study on the internalization rate of hRS7-VK-PTX

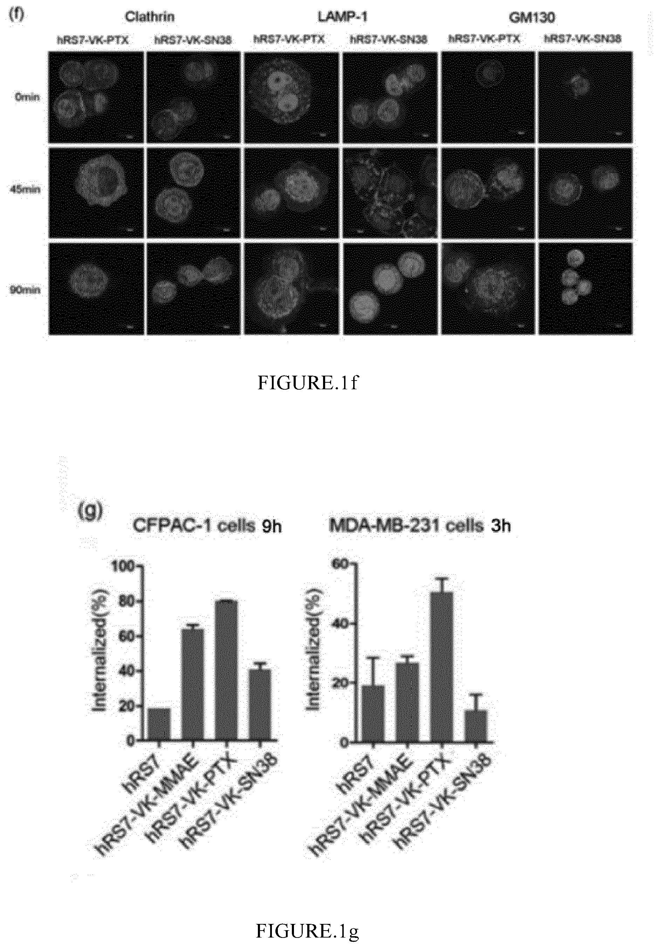

The internalization rate of ADC may affect its inhibitory efficiency against tumor cells. Interestingly, the internalization rate of hRS7-VK-PTX is faster than hRS7-VK-MMAE and hRS7-VK-SN38 in Trop-2 expressing cells (Fig. 1g).

SKBR3 cells were incubated with ADC molecules at 37 °C for 45 or 90 min. Unbound ADC was removed by washing cells in a culture medium, then the cells were fixed and permeabilized. The marker proteins LAMP-1, GM130 and clathrin were detected with their respective antibodies and a secondary antibody conjugated to Alex^{®}555 (red). Anti-Trop-2 ADC was detected with DyLight^{®}488-conjugated secondary antibody (green). DAPI was used to stain nuclear DNA (blue). Confocal results showed the co-localization of hRS7-VK-PTX molecule with lysosomes and clathrin heavy chain, suggesting that it was internalized through clathrin-mediated endocytosis and transported into the lysosomal compartment. In contrast, hRS7-VK-SN38 did not undergo significant internalization (Fig. 1f).

These results show that hRS7-VK-PTX can trigger a faster internalization rate, which provides support for a higher cell killing potency.

### Example 7 Inhibitory effect of hRS7-VK-PTX on tumor cells specifically expressing Trop-2 in vivo and "bystander effect"

### I. Inhibitory effect of hRS7-VK-PTX on tumor cells specifically expressing Trop-2 in vivo

Mice inoculated with COLO205 cells (n = 6 per group) were treated with ADC or control drug on days 7, 11, 15, and 19, and tumor volume was measured two or three times a week. Statistical significance between treatment and control groups was assessed using a two-tailed t-test. ***P<0.001, comparing hRS7-VK-PTX with hRS7; p<0.001, comparing hRS7-VK-PTX with hRS7-VK-SN38, data = mean ± SD, the results are shown in Fig. 1c.

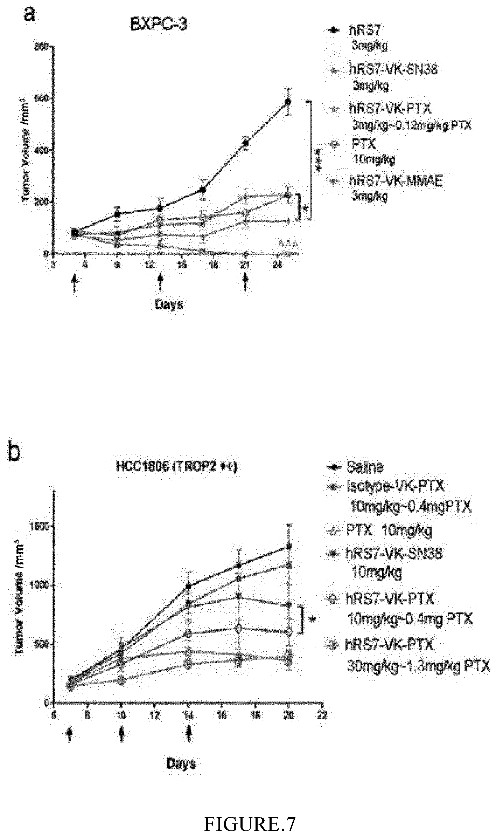

Mice inoculated with BXPC-3 cells (n = 5 per group) were treated with ADC or naked antibody on days 5, 13, and 21, and tumors were measured two or three times a week. On day 25, statistical significance between treatment and control groups was assessed using a two-tailed t-test. ***P<0.001, compared with hRS7 treatment group; *P<0.05, comparing hRS7-VK-PTX treatment group with PTX treatment group; compared with hRS7 treatment group, P<0.001, data=mean ± SD, the results are shown in Figure 7a.

Mice inoculated with HCC1806 cells (n = 6 per group) were treated on days 7, 10 and 14. Tumors were measured two or three times per week. On day 20, statistical significance between treatment and control groups was assessed using a two-tailed t-test. *P <0.05, comparing hRS7-VK-PTX treatment group with hRS7-VK-SN38 treatment group, data = mean ± SD, the results are shown in Figure 7b.

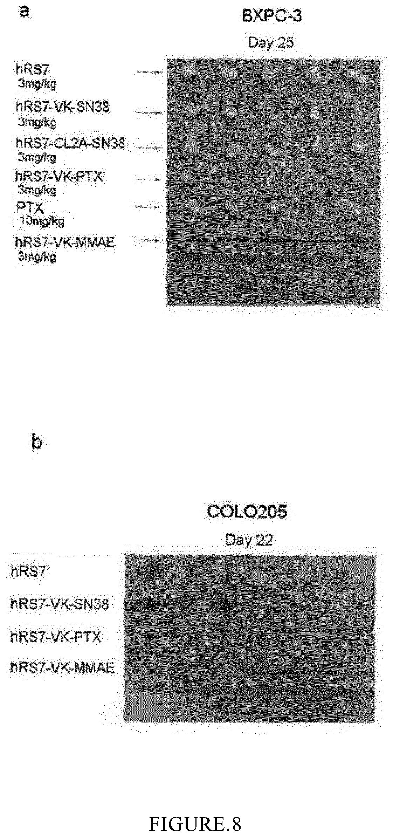

Tumor tissues dissected from mouse transplanted tumor models treated with different ADC molecules were compared. ADC treatment inhibited tumor growth in BXPC3 (a) or COLO205 cell-derived (b) mouse xenograft models. Tumor tissues were collected on day 25 (Figure 8a) or day 22 (Figure 8b), "-" indicates complete inhibition.

By examining the therapeutic potential of hRS7-VK-PTX in a mouse xenograft tumor model, it was found that hRS7-VK-PTX was more effective than hRS7-VK-SN38 in inhibiting BXPC-3, COLO205, and HCC1806 tumor cells in a xenograft tumor model (Fig. 1c and Fig. 7, Fig. 8).

Furthermore, 3 mg/kg of hRS7-VK-PTX was more effective than 10 mg/kg of PTX in inhibiting BxPC-3 transplanted tumor model (P = 0.0248) (Fig. 7a).

In the HCC1806 cell transplanted tumor model, the tumor inhibitory effect of hRS7-VK-PTX at a dose of 30 mg/kg (1.3 mg/kg PTX equivalent) was comparable to that of 10 mg/kg of PTX (Fig. 7b).

These results indicates that when PTX is prepared to the form of ADC using hydrophilic linkers, the anti-tumor effect of PTX in vivo can be significantly improved.

### II. "Bystander effect" of hRS7-VK-PTX in mouse transplanted tumor model

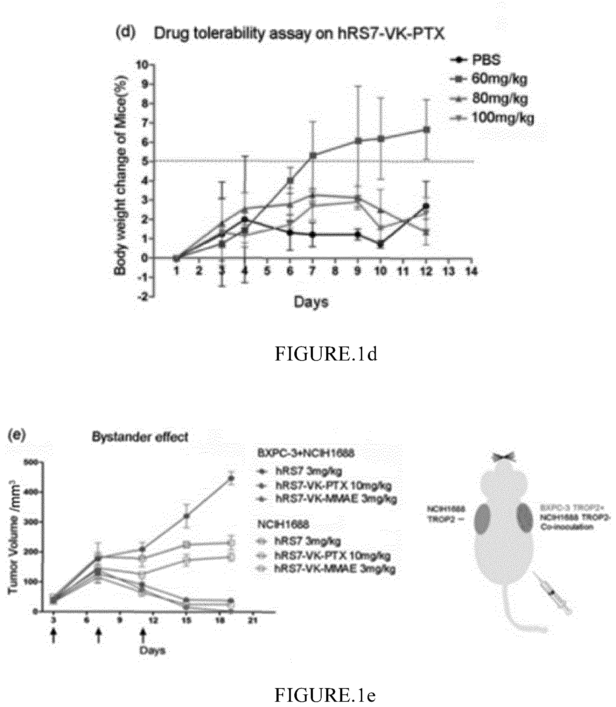

A mixture of Trop-2-positive tumor cells BXPC-3 and Trop-2-negative tumor cells NCIH1688 or NCIH1688 cells alone were inoculated into BALB/c-nu/nu mice (Fig. 1e, right panel), and the "bystander effect" of hRS7-VK-PTX was detected in a mouse transplanted tumor model.

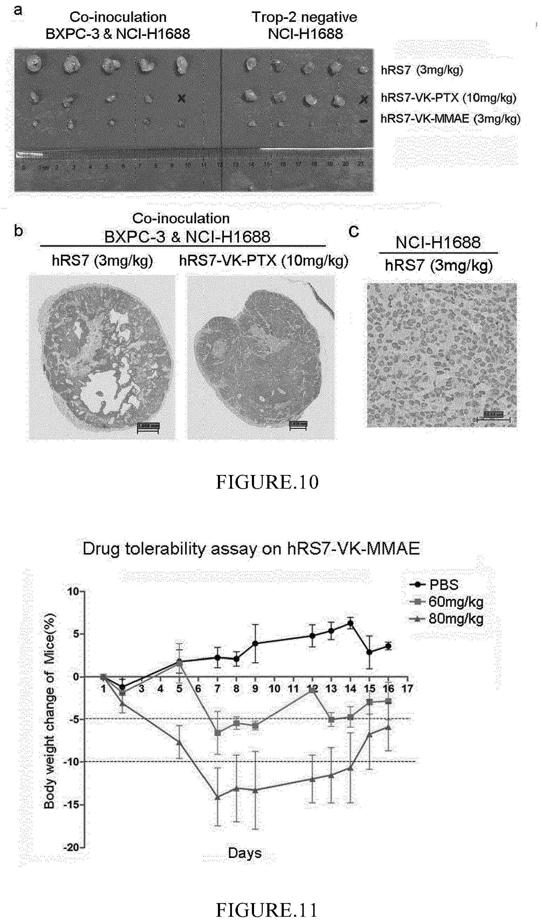

Immunohistochemical staining revealed heterogeneity of Trop-2 expression in transplanted tumor derived from mixed cells and loss of Trop-2 expression in transplanted tumor derived from NCI-H1688 cells alone (Fig. 10b and c). Although PTX as a small molecule is far less potent than MMAE, treatment with both hRS7-VK-PTX (10 mg/kg) and hRS7-VK-MMAE (3 mg/kg) significantly inhibited the growth of transplanted tumors heterogeneously expressing Trop-2, and tumor growth inhibition index (TGI) were 99.5% and 91.7%, respectively (Figure 1e and Figure 10a).

In addition, hRS7-VK-MMAE also had a killing effect on transplanted tumors inoculated with NCIH1688 cells not expressing Trop-2 alone, but hRS7-VK-PTX had no such effect (Fig. 1e and Fig. 10a).

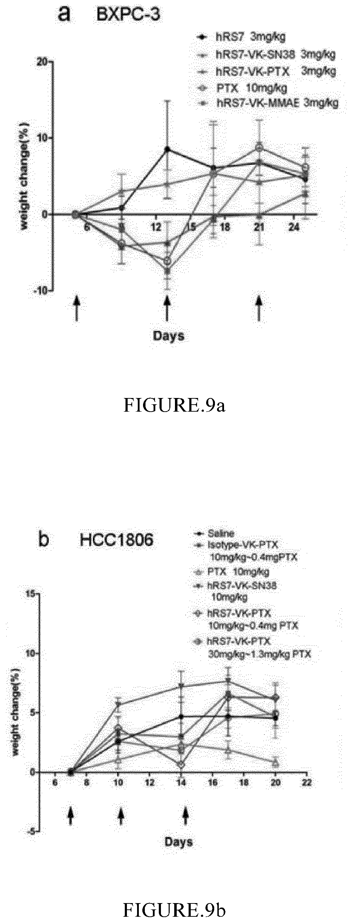

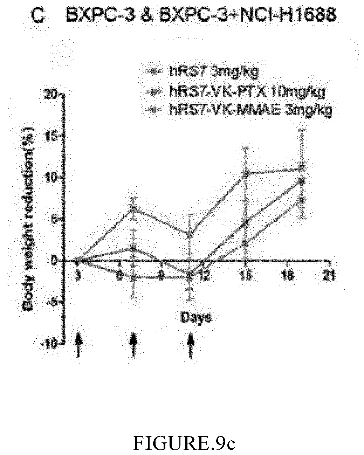

The above results show that hRS7-VK-PTX has "bystander effect", and also show that the cell killing effect of hRS7-VK-PTX is more Trop-2 specific than hRS7-VK-MMAE, which will be a reliable evidence for the safety in clinical application. Furthermore, hRS7-VK-PTX (3 or 10 mg/kg) did not cause adverse effects or weight loss in treated mice (Fig. 9), which is in stark contrast to the treatment groups with hRS7-VK-MMAE(3 mg / kg), hRS7-VK-SN38(3 or 10 mg / kg) or PTX alone.

### Example 8 Tolerability Study of hRS7-VK-PTX

The safety of relatively high doses of hRS7-VK-PTX in BALB/c mice was studied by an acute toxicity experiment with single intravenous administration in mice. Although the maximum tolerated dose (MTD) of hRS7-VK-PTX was not reached due to limited reagent availability, it showed no significant toxicity or weight loss compared with placebo at a single 100 mg/kg dose (Fig. 1d). But compared with hRS7-VK-MMAE, hRS7-VK-MMAE at only 60 mg/kg caused severe adverse reactions and more than 15% body weight loss in mice (Fig. 11).

Therefore, hRS7-VK-PTX has better safety and tolerability than hRS7-VK-MMAE.

All documents mentioned in this application are incorporated by reference in this application as if each were individually incorporated by reference. In addition, it should be understood that after reading the above teaching content of the present invention, those skilled in the art can make various changes or modifications to the present invention, and these equivalent forms also fall within the scope defined by the appended claims of the present application.

## Claims

1. A compound of formula I, 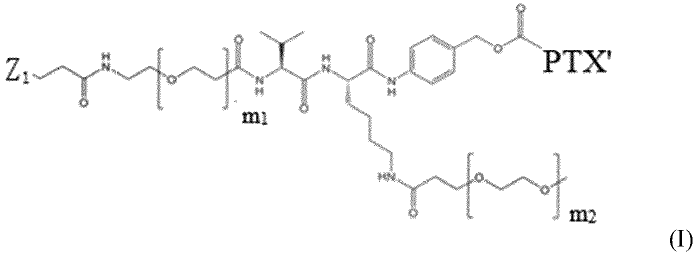 in the formula,
Z₁ is a linker moiety for linking with an antibody;
m₁ is an integer of 0-10;
m₂ is an integer of 10-50;
PTX' is a taxoid compound.

2. The compound of formula I according to claim 1, wherein the structure of the compound of formula I is as shown in formula Ia: 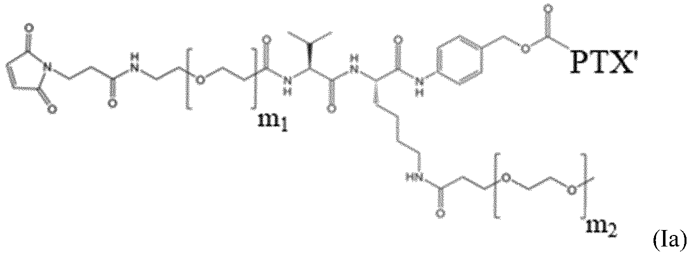 in the formula,
m₁ is an integer of 0-10;
m₂ is an integer of 10-50;
PTX' is a taxoid compound.

3. The compound of formula I according to claim 1, wherein the structure of the compound of formula I is as shown in formula III: 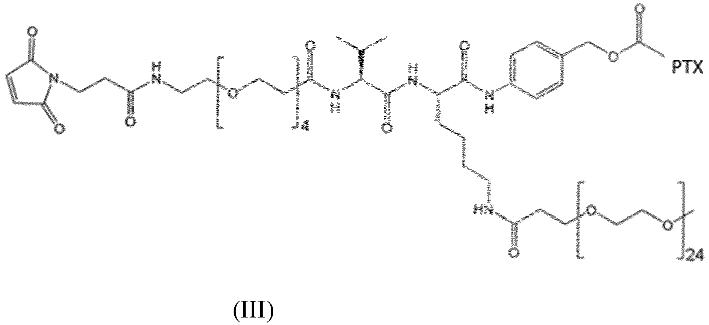 where, PTX is paclitaxel.

4. An antibody-drug conjugate (ADC), wherein the antibody-drug conjugate is an antibody-drug conjugate (ADC) formed by coupling the compound of formula I of claim 1 with an antibody.

5. The antibody-drug conjugate according to claim 4, wherein the antibody-drug conjugate has the structure shown in Formula II:
Ab-(L-PTX')n II
in the formula,
Ab represents antibody,
L is 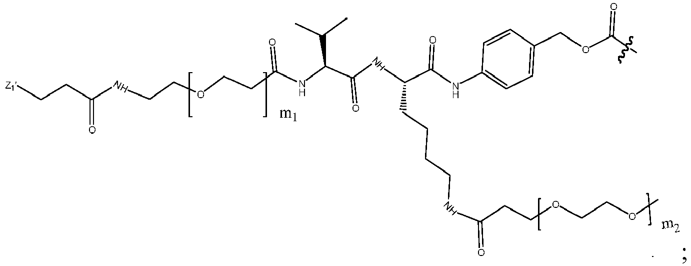
wherein, Z₁' is a group formed from Z₁ group by removing a hydrogen atom;
PTX' is a taxoid compound;
n is the average coupling number of the drug coupled to the antibody;
m₁ and m₂ are as defined in claim 1.

6. The antibody-drug conjugate according to claim 4, wherein the antibody is selected from the following group consisting of: chimeric antibodies, bivalent or bispecific molecules, bispecific antibodies, trispecific antibodies and tetra-specific antibodies.

7. The antibody-drug conjugate according to claim 4, wherein the antibody is selected from the following group consisting of:
Trop-2, PD-L1, CTLA4, OX40, EpCAM, EGFR, EGFRVIII, HER2, PTPN2, VEGF, CD11a, CD19, CD20, CD22, CD25, CD30, CD33, CD40, CD56, CD64, CD70, CD73, CD74, CD79, CD105, CD138, CD174, CD205, CD227, CD326, CD340, MUC16, GPNMB, PSMA, Cripto, ED-B, TMEFF2, EphB2, Apo-2, NMP179, p16INK4A, Nectin-4, B7H3, EMA, CEA, MIB-1, GD2, APRIL receptor, folate receptor, mesothelin inhibitor, MET and TM4SF1.

8. The antibody-drug conjugate according to claim 4, wherein the antibody is hRS7.

9. A pharmaceutical composition, wherein the pharmaceutical composition comprises: (a) the antibody-drug conjugate of claim 4, and (b) a pharmaceutically acceptable carrier.

10. The antibody-drug conjugate of claim 4 or the pharmaceutical composition of claim 9 for use in treating tumors or immune diseases.

11. A method for preparing the antibody-drug conjugate of claim 4, wherein the method comprises the following steps:
(1) providing a reaction system, the reaction system includes the antibody to be coupled and the compound of formula I of claim 1;
(2) in the reaction system, the antibody is coupled to the compound of formula I to produce the antibody-drug conjugate of claim 4.

12. A compound of formula IV 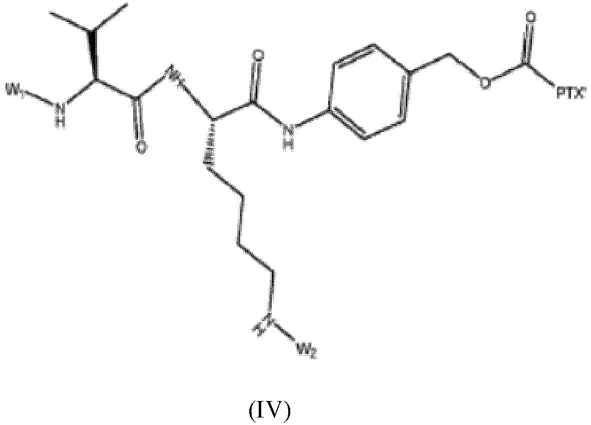 in the formula, W₁ is selected from: H, Fmoc-, 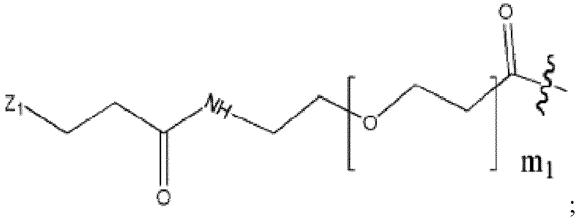
W₂ is selected from: H, Trt-, 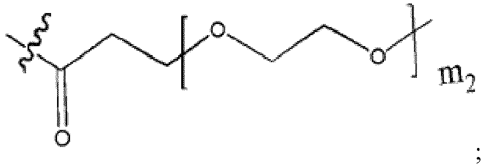
PTX', m₁, m₂ and Z₁ are as defined in claim 1.

13. Use of the compound of formula I of claim 1 in the preparation of an antibody-drug conjugate.
